# FASCICULE DE BREVET EUROPEEN

(11) **EP 0 994 102 B1**
(45) Date de publication et mention de la délivrance du brevet: **19.03.2003**
(21) Numéro de dépôt: 99402495.8
(22) Date de dépôt: 12.10.1999
(51) Int. Cl.: C07C 233/23, C07C 233/18, C07D 307/81, C07D 333/58, C07D 311/92, C07D 471/04, A61K 31/16, A61K 31/38, A61K 31/335, A61K 31/395

(54) **Dérivés cycliques à chaîne cycloalkylénique, leur procédé de préparation et les compositions pharmaceutiques qui les contiennent**
Zyklische Derivate mit einer zykloalkylenischen Kette, Verfahren zu ihrer Herstellung und pharmazeutische Zusammensetzungen die diese enthalten
Cyclic derivatives with a cycloalkylenic chain, process for their preparation and pharmaceutical compositions containing them

(30) Priorité: 12.10.1998 FR 9812738
(43) Date de publication de la demande: 19.04.2000
(73) Titulaire: LES LABORATOIRES SERVIER, 92200 Neuilly sur Seine (FR)
(72) Inventeur: Langlois, Michel, 92330 Sceaux (FR); Mathe-Allainmat, Monique, 91300 Massy (FR); Lefas-Le Gall, Marie, 95170 Deuil La Barre (FR); Bennejean, Caroline, 94220 Charenton Le Pont (FR); Renard, Pierre, 78150 Le Chesnay (FR); Delagrange, Philippe, 92130 Issy Les Moulineaux (FR)

(56) Documents cités:
- EP-A- 0 745 583
- EP-A- 0 745 584
- EP-A- 0 747 346
- WO-A-97/06140

## Description

La présente invention concerne de nouveaux dérivés cycliques à chaîne cycloalkylénique, leur procédé de préparation et les compositions pharmaceutiques qui les contiennent.

On connaît, dans l'art antérieur des hydroxy ou alkoxy amides cyclopropaniques (WO 9209566, EP 436199, US 5459150) et des hydroxy ou alkoxy amides cyclobutaniques (US 5187192) utiles en tant qu'inhibiteurs de 5-lipoxygénase.

D'autres dérivés amides ou thioamides cyclopropaniques à chaîne insaturée sont décrits en tant que pesticides (EP 369762).

On trouve également des composés indoliques cyclopropaniques utiles dans le traitement des maladies neurodégénératives (EP 568136).

Enfin, des publications mentionnent des composés amides à chaîne cyclohexanique en tant qu'intermédiaires ou produits de synthèse (Indian J. Chem., 1974, 12(7), pp. 664-7 ; Bull. Chem. Soc. Jap., 1968,41(12), pp. 3008-11).

Les composés de la présente invention sont nouveaux et présentent des caractéristiques pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.
Outre leur action bénéfique sur les troubles du rythme circadien (J. Neurosurg. 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement des maladies de Parkinson (J. Neurosurg. 1985, 63, pp 321-341) et d'Alzheimer (Brain Research, 1990, 528, pp 170-174). De même, ces composés ont montré une activité sur certains cancers (Melatonin - Clinical Perspectives, Oxford University Press, 1988, pp 164-165), sur l'ovulation (Science 1987, 227, pp 714-720), sur le diabète (Clinical Endocrinology, 1986, 24, pp 359-364), et dans le traitement de l'obésité (International Journal of Eating Disorders, 1996, 20 (4), pp 443-446).
Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention outre leur nouveauté, montrent une très forte affinité pour les récepteurs de la mélatonine et/ou une sélectivité pour l'un ou l'autre des sous-types réceptoriels mélatoninergiques.

La présente invention concerne plus particulièrement les composés de formule (I) : 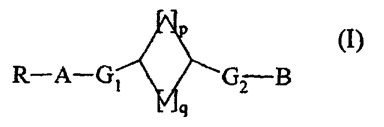 dans laquelle
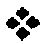 A représente :
   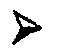 un système cyclique de formule (II) : 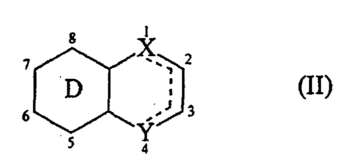 où X et Y, identiques ou différents, représentent un atome d'oxygène, un atome de soufre ou un groupement CH₂,
      D représente un cycle benzénique ou un naphtalène,
      et la représentation 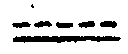 signifie que les liaisons peuvent être simples ou doubles, étant entendu que la valence des atomes est respectée,
      dans lequel R substitue le système cyclique D ou le cycle contenant X et Y, et G₁ substitue le cycle contenant X et Y,
   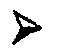 ou un système cyclique de formule (III) : 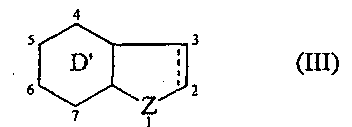 où Z représente un atome d'oxygène, un atome de soufre, un groupement CH₂, ou un groupement NR¹ (dans lequel R¹ représente un atome d'hydrogène, un groupement alkyle (C₁-C₆) linéaire ou ramifié, aryle, ou arylalkyle (C₁-C₆) linéaire ou ramifié),
      D' représente un cycle benzénique ou un cycle pyridinique, et la représentation 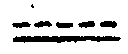 a la même signification que précédemment,
      dans lequel R substitue le cycle D' et G₁ substitue l'autre cycle,
      étant entendu que les systèmes cycliques de formule (II) ou (III) peuvent être substitués, en plus des groupements R et G₁, par un à trois groupements, identiques ou différents, choisis parmi Rₐ, ORₐ, hydroxyle, CORₐ, formyle, COORₐ, carboxyle ou OCORₐ, où Rₐ représente un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, polyhalogénoalkyle (C₁-C₆) linéaire ou ramifié, cycloalkyle (C₃-C₈) substitué ou non, cycloalkyle (C₃-C₈) alkyle (C₁-C₆) linéaire ou ramifié substitué ou non, cycloalkényle (C₃-C₈) substitué ou non, cycloalkényle (C₃-C₈) alkyle (C₁-C₆) linéaire ou ramifié substitué ou non, aryle, arylalkyle (C₁-C₆) linéaire ou ramifié, hétéroaryle ou hétéroarylalkyle (C₁-C₆) linéaire ou ramifié,
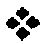 R représente un atome d'hydrogène ou d'halogène, un groupement hydroxyle, SH, Rₐ, ORₐ ou S(O)ₙRₐ où n vaut 0, 1 ou 2 et Rₐ est tel que défini précédemment,
   ou forme, avec l'atome de carbone qui le porte et un atome de carbone adjacent un cycle de formule (IV) 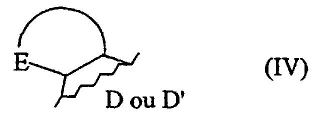 dans laquelle E représente un atome d'oxygène ou un groupement -S(O)ₙ- où n est tel que défini précédemment, le cycle ainsi formé contenant de 5 à 7 atomes, pouvant contenir une ou plusieurs insaturations et pouvant être substitué par un ou plusieurs groupements choisis parmi alkyle (C₁-C₆) linéaire ou ramifié, alkoxy (C₁-C₆) linéaire ou ramifié, carboxy, alkoxycarbonyle (C₁-C₆) linéaire ou ramifié, hydroxy ou oxo,
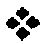 G₁ et G₂, identiques ou différents, représentent une liaison simple ou une chaîne alkylène -(CH₂)ₜ- (dans laquelle t vaut 1, 2, 3 ou 4), substituée ou non par un ou plusieurs radicaux, identiques ou différents, choisis parmi hydroxy, carboxy, formyl, Rₐ, ORₐ, COORₐ, CORₐ, (dans lesquels Rₐ est tel que défini précédemment) ou atomes d'halogène,
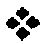 p vaut 0, 1, 2, 3 ou 4,
   q vaut 0, 1, 2, 3 ou 4,
   avec 1 ≤ p+q ≤ 4,
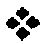 B représente un groupement -NR¹ₐC(Q)R²ₐ, -NR¹ₐC(Q)NR²ₐR³ₐ, ou -C(Q)NR¹ₐR²ₐ dans lesquels R¹ₐ, R²ₐ et R³ₐ, identiques ou différents, peuvent prendre toutes les valeurs de Rₐ et peuvent représenter un atome d'hydrogène, et Q représente un atome d'oxygène ou de soufre,
étant entendu que :
- le terme "substitué" affecté aux expressions "alkyle", "alkényle", "alkynyle", signifie que ces groupements sont substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
- le terme "substitué" affecté aux expressions "cycloalkyle", "cycloalkylalkyle", "cycloalkényle", "cycloalkénylalkyle", signifie que la partie cyclique de ces groupements est substituée par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
- par "aryle" on entend les groupements phényle, naphtyle ou biphényle, ces groupements pouvant être substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, cyano, carboxy, alkoxy (C₁-C₆) linéaire ou ramifié carbonyle, nitro, amino ou atomes d'halogène,
- par "hétéroaryle" on entend tout groupement aromatique mono ou polycyclique contenant 5 à 10 atomes contenant 1 à 3 hétéroatomes choisis parmi azote, oxygène ou soufre, ces groupements pouvant être substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, cyano, carboxy, alkoxy (C₁-C₆) linéaire ou ramifié carbonyle, nitro, amino ou atomes d'halogène,
étant entendu que :
- lorsque A représente un noyau naphtalène non substitué ou substitué par un groupement méthoxy ou par un groupement méthyle, G₁ et G₂ représentent simultanément une simple liaison, et B représente un groupement 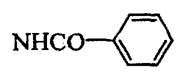 ou NHCOMe, alors on ne peut avoir (q = 0 et p = 4) ou (q = 4 et p = 0),
- lorsque G₁ représente une simple liaison et p+q=1, alors A ne peut représenter un noyau naphtalène substitué par un ou plusieurs atomes d'halogène,
- lorsque A représente un noyau indolique substitué en position 2 par le groupement 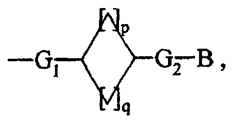 alors B ne peut représenter un groupement urée,
- lorsque A représente un noyau indolique substitué en position 2 par un groupement carboxyle ou alkoxycarbonyle, p vaut 1 et q vaut 0 (ou q vaut 1 et p vaut 0), et G₁ représente une liaison simple, alors B ne peut représenter un groupement CONHAr dans lequel Ar représente un groupement aryle,
leurs énantiomères et diastéréoisomères, ainsi que leurs sels d'addition à un acide ou à une base pharmaceutiquement acceptable.

Parmi les acides pharmaceutiquement acceptables, on peut citer les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique.

Parmi les bases pharmaceutiquement acceptables, on peut citer l'hydroxyde de sodium, l'hydroxyde de potassium, la triéthylamine, la tertbutylamine.

Les composés préférés de l'invention sont ceux pour lesquels p+q=1.

Les substituants R préférés sont les groupements Rₐ, ORₐ et SRₐ, plus particulièrement Rₐ et ORₐ dans lesquels Rₐ représente plus préférentiellement un groupement alkyle (C₁-C₆) linéaire ou ramifié, cycloalkyle (C₃-C₈), cycloalkyle (C₃-C₈) alkyle (C₁-C₆) linéaire ou ramifié, aryle ou arylalkyle (C₁-C₆) linéaire ou ramifié, comme par exemple méthyle, éthyle, propyle, isopropyle, butyle, sec-butyle, tert-butyle, pentyle, hexyle, cyclopropyle, cyclobutyle, cyclopentyle, cyclohexyle, cyclopropylméthyle, cyclopropyléthyle, phényle
ou benzyle,
ou ceux pour lesquels R forme avec l'atome de carbone qui le porte et un atome de carbone adjacent un cycle de formule (IV) dans lequel E représente par exemple -Q-(CH₂)₂-, -Q-(CH₂)₃-, -Q-CH=CH-, -Q-CH₂-CH=CH-, 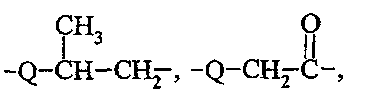 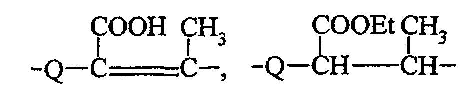 où Q représente un atome de soufre ou d'oxygène.

L'invention concerne encore plus préférentiellement les composés pour lesquels R représente un groupement ORₐ.

De façon plus avantageuse, l'invention concerne les composés pour lesquels A représente un système cyclique de formule (II) substitué en position 7 par R et en 1 ou 2 par G₁, ou un système de formule (III) substitué en position 5 par R et en position 3 par G₁, A étant préférentiellement non substitué ou substitué (en plus des substituants R et G₁) par un groupement en position 2 ou 3 (formule II) ou 2 (formule III), ce groupement étant plus préférentiellement un groupement alkoxy, aryle ou arylalkyle.

Les groupements G₁ et G₂ préférés sont la liaison simple ou le groupement CH₂.

L'invention concerne plus particulièrement les composés pour lesquels B représente un groupement -NHCORₐ ou -CONHRₐ.

De façon encore plus avantageuse, l'invention concerne les composés pour lesquels A est substitué :
- par un groupement de formule (V) 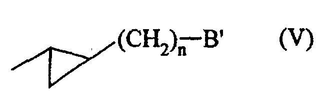 dans laquelle n vaut 0 ou 1 et B' représente un groupement -NHCORₐ ou -CONHRₐ (où Rₐ tel que défini précédemment),
- par un groupement alkoxy, alkylthio ou alkyle,
- et optionnellement par un groupement alkoxy, aryle ou arylalkyle.

Encore plus particulièrement, l'invention concerne :
les composés naphtaléniques, chromaniques ou benzochromaniques substitués en 1 ou 2 (formule II) par un groupement de formule (V), en 7 (formule II) par un groupement Rₐ, ORₐ ou SRₐ, et optionnellement substitués en 3 (formule II) par un groupement aryle ou arylalkyle ou en 2 (formule II) par un groupement alkoxy,
ou les composés benzothiophéniques, benzofuraniques, indoliques ou azaindoliques substitués en 3 (formule III) par un groupement de formule (V), en 5 (formule III) par un groupement Rₐ, ORₐ ou SRₐ et optionnellement substitués en 2 (formule III) par un groupement aryle ou arylalkyle.

Très avantageusement, l'invention concerne les dérivés naphtaléniques substitués en position 1 par un groupement de formule (V), en position 7 par un groupement ORₐ et optionnellement substitués en position 2 par un groupement ORₐ ou en position 3 par un groupement aryle ou arylalkyle.

L'invention concerne tout particulièrement les composés de formule (I) qui sont
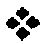 N-[2-(7-méthoxy-1-naphtyl)-1-cyclopropyl]acétamide
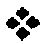 N-[2-(7-méthoxy-1-naphtyl)-1-cyclopropyl]propanamide
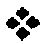 N-[2-(7-méthoxy-1-naphtyl)-1-cyclopropyl]butanamide
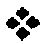 N-[2-(7-méthoxy-1-naphtyl)-1-cyclopropyl]cyclopropane carboxamide
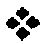 N-[2-(2,7-diméthoxy-1-naphtyl)-1-cyclopropyl]acétamide
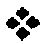 N-[2-(2,7-diméthoxy-1-naphtyl)-1-cyclopropyl]propanamide
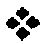 N-[2-(2,7-diméthoxy-1-naphtyl)-1-cyclopropyl]butanamide
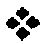 N-[2-(2-méthoxy-1-naphtyl)-1-cyclopropyl]acétamide
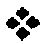 N-[2-(2-méthoxy-1-naphtyl)-1-cyclopropyl]propanamide
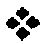 N-[2-(2-méthoxy-1-naphtyl)-1-cyclopropyl]butanamide
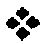 N-[2-(2-méthoxy-1-naphtyl)-1-cyclopropylméthyl]acétamide
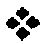 N-[2-(2-méthoxy-1-naphtyl)-1-cyclopropylméthyl]propanamide
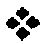 N-[2-(2-méthoxy-1-naphtyl)-1-cyclopropylméthyl]butanamide.

La configuration préférée des composés de formule (I) est celle pour laquelle le groupement -G₁-A-R est en *trans* par rapport au groupement -G₂-B.

Les énantiomères, diastéréoisomères ainsi que les sels d'addition à un acide ou une base pharmaceutiquement acceptable des composés préférés de l'invention font partie intégrante de l'invention.
L'invention s'étend également au procédé de préparation des composés de formule (I) caractérisé en ce que l'on utilise comme produit de départ le composé de formule (VI) : 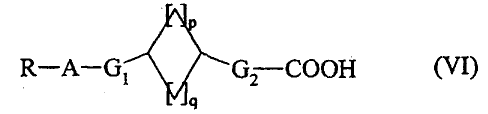 dans laquelle R, A, G₁, G₂, p et q sont tels que définis précédemment,
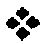 qui est soumis, après activation sous forme de chlorure d'acide ou en présence d'agent de couplage, à l'action d'une amine HNR¹ₐR²ₐ, dans laquelle R¹ₐ et R²ₐ sont tels que définis précédemment pour conduire au composé de formule (I/a), cas particulier des composés de formule (I) : 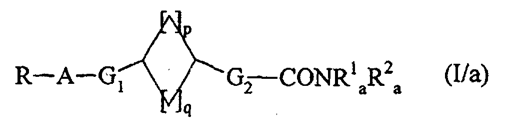 dans laquelle R, A, G₁, G₂, R¹ₐ, R²ₐ, p et q sont tels que définis précédemment, qui peut être soumis à un agent de thionation comme le réactif de Lawesson pour obtenir le composé de formule (I/b), cas particulier des composés de formule (I), 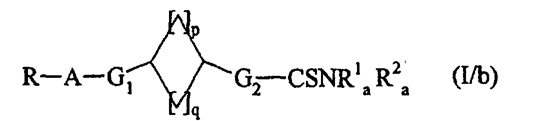 dans laquelle R, A, G₁, G₂, R¹ₐ, R²ₐ, p et q sont définis comme précédemment,
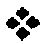 ou soumis, après transformation en azidure correspondant à un réarrangement de Curtius pour conduire après hydrolyse à l'amine de formule (VII) 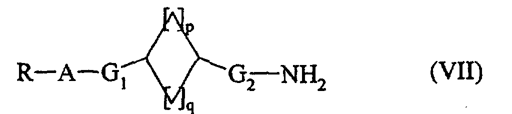 dans laquelle R, A, G₁, G₂, p et q sont tels que définis précédemment, qui est :
   - soit mise en réaction avec un chlorure d'acyle ClCOR¹ₐ ou l'anhydride mixte ou symétrique correspondant pour lesquels R¹ₐ est défini comme précédemment pour conduire au composé de formule (I/c), cas particulier des composés de formule (I) : 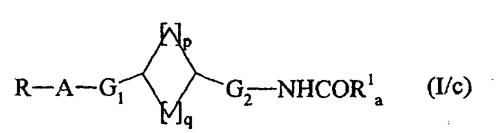 dans laquelle R, A, G₁, G₂, R¹ₐ, p et q sont définis comme précédemment, suivi éventuellement de l'action d'un composé de formule (VIII)

      Rₐ―J (VIII)

      dans laquelle Rₐ est tel que défini précédemment et représente un groupe partant comme un atome d'halogène ou un groupement tosyle,
   pour conduire au composé de formule (I/d), cas particulier des composés de formule (I) : 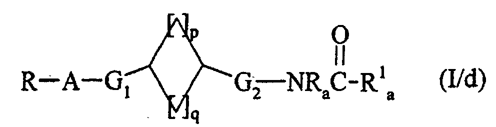 dans laquelle R, A, G₁, G₂, Rₐ, R¹ₐ, p et q sont tels que définis précédemment,
   l'ensemble des composés (I/c) et (I/d) formant le composé de formule (I/e), cas particulier des composés de formule (I) : 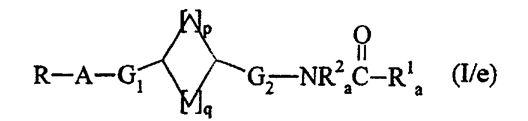 dans laquelle R, A, G₁, G₂, R¹ₐ, R²ₐ, p et q sont tels que définis précédemment,
   composé de formule (I/e) qui peut être soumis à un agent de thionation comme le réactif de Lawesson par exemple pour conduire au composé de formule (I/f), cas particulier des composés de formule (I) : 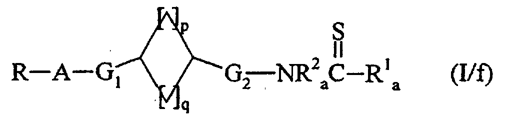 dans laquelle R, A, G₁, G₂, R¹ₐ, R²ₐ, p et q sont tels que définis précédemment,
   - soit soumise à l'action d'un composé de formule (IX) :

      Q=C=N-R¹ₐ (IX)

      dans laquelle Q et R¹ₐ sont tels que définis précédemment,
      suivi éventuellement de l'action d'un composé de formule (VIII) pour conduire au composé de formule (I/g), cas particulier des composés de formule (I) : 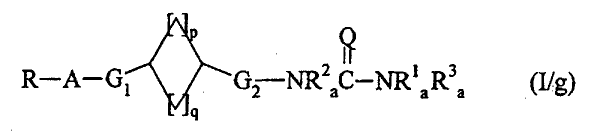
   dans laquelle R, A, G₁, G₂, R¹ₐ, R²ₐ, R³ₐ, Q, p et q sont définis comme précédemment,
les composés (Va) à (I/g) formant l'ensemble des composés de formule (I) et pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

Les composés (VI) de départ sont soit commerciaux, soit aisément accessibles à l'homme du métier par des réactions chimiques classiques ou décrites dans la littérature.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils étaient atoxiques, doués d'une très haute affinité sélective pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation, qui permettent d'établir que les produits de l'invention sont utiles dans le traitement du stress, des troubles du sommeil, de l'anxiété, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisées dans les dysfonctionnements sexuels, qu'ils possèdent des propriétés d'inhibiteurs de l'ovulation, d'immunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en 1 ou plusieurs prises.

Les exemples suivants illustrent l'invention et ne la limitent en aucune façon. Les préparations suivantes conduisent à des intermédiaires de synthèse utiles dans la préparation des composés de l'invention.

### Préparation 1 : Trans 2-(7-méthoxy-1-naphtyl)-1-cyclopropanamine

### Stade A : (7-Méthoxy-1-naphtyl)méthyl acétate

Dans un ballon de 2 litres, 48,6 g (0,225 mol) d'acide [7-méthoxy (napht-1-yl)] acétique sont solubilisés dans un mélange Benzène 700 ml / Acide acétique 300 ml et portés au reflux sous argon afin d'être dégazés. A température ambiante 100 g (1 eq) de tétracétate de plomb sont ajoutés. Le mélange réactionnel est chauffé à 60-70°C, température à laquelle intervient un fort dégagement gazeux (départ de CO₂), puis 30 minutes à ébullition. Le milieu réactionnel est concentré sous vide, repris à chaud au CH₂Cl₂ (200 ml); sous agitation 500 ml d'éther sont ajoutés lentement. L'important précipité blanc crème qui se forme, est filtré sur célite et rincé à l'éther. La phase éthérée est alors basifiée à l'aide d'une solution glacée de bicarbonate de sodium à 5 %, neutralisée à l'eau, séchée sur Na₂SO₄ et concentrée sous pression réduite pour conduire à une huile qui recristallise lentement à froid.

### Stade B : (7-Méthoxy-1-naphtyl)méthanol

34,5 g (0,15 mol) du composé obtenu au stade A sont dissous dans 300 ml de méthanol. Sous forte agitation, 35 g (4 eq) d'hydroxyde de potassium solubilisés dans 35 ml d'eau sont additionnés. Après 3 heures à température ambiante, le mélange réactionnel est hydrolysé sur un mélange glace/acide chlorhydrique concentré. Le précipité blanc crème qui se forme, est filtré, lavé plusieurs fois à l'eau glacée et séché au dessicateur sur P₂O₅. L'alcool du titre est recristallisé dans un mélange CH₂Cl₂/cyclohexane pour donner des aiguilles blanches.

### Stade C : 7-Méthoxy-1-naphtaldéhyde

19,2 g (0,1 mol) du composé obtenu au stade B sont solubilisés dans 300 ml de CH₂Cl₂. Sous argon, 65 g (7,5 eq) d'oxyde de manganèse sont ajoutés en trois fois (t = 0 : 25 g, t = 3h : 25g, et t = 24h : 15g). L'alcool est totalement oxydé après 24 heures supplémentaires. Le mélange est alors filtré sur célite afin d'éliminer les composés minéraux, rincé au CH₂Cl₂ puis filtré sur silice pour conduire à une huile se solidifiant par refroidissement.

### Stade D : 3-(7-Méthoxy-1-naphtyl)-2-propénoate d'éthyle

2 g (10,7 mmol) du composé obtenu au stade C dans 20 ml de THF anhydre sont mis en présence de 515 mg (1,2 eq) de NaH (60 % dans l'huile) dans 25 ml de THF anhydre et de 2,5 ml (1,2 eq) de triéthylphosphonoacétate. Après 3 heures d'agitation à température ambiante et une nuit au léger reflux, on obtient une huile foncée.

### Stade E : Acide 3-(7-méthoxy-1-naphtyl)-2-propénoïque

8,4g (32,8 mmol) de l'ester obtenu au stade D solubilisé dans 150 ml d'éthanol en présence de 40 ml de soude 2N sont agités 3 heures à température ambiante et la nuit au reflux pour conduire à l'acide du titre sous forme d'un solide blanc.

### Stade F : N-Méthoxy-N-méthyl-3-(7-méthoxy-1-naphtyl)-2-propènamide

Le chlorure d'acide obtenu à partir de 6,5 g (28,5 mmol) de l'acide obtenu au stade E et 5 ml de chlorure d'oxalyle est additionné goutte à goutte sur la suspension de chlorure de N,O-diméthylhydroxylamine (5,6 g) dans un mélange CH₂Cl₂/H₂O en présence de 3 g de Na₂CO₃. L'acide protégé est obtenu sous forme d'un solide jaune pâle .

### Stade G : Trans N-méthoxy-N-méthyl-2-(7-méthoxy-1-naphtyl)-1-cyclopropane carboxamide

A une suspension d'ylure généré à partir de 10,6 g (2 eq) de Me₃SOI solubilisés à chaud dans 50 ml de DMSO et de 1,25 g de NaH, 5,8 g (21,4 mmol) du dérivé obtenu au stade F solubilisés dans 30 ml de DMSO sont additionnés. Après 15 heures d'agitation à température ambiante et 4 heures à 50°C l'amide cyclopropanique du titre est obtenue sous forme d'une huile marron.

### Stade H : Acide trans 2 - (7-méthoxy-1-naphtyl)-1-cyclopropane carboxylique

6 g (21 mmol) du composé obtenu au stade G dans 50 ml d'éther anhydre et 15,4 g de tertiobutylate de potassium sont agités 2 jours à température ambiante. 3,2 g d'un solide marron clair sont recueillis et mis en réaction sans purification.

### Stade I : Trans 2-(7-méthoxy-1-naphtyl)-1-cyclopropanamine

1,5 g (6,2 mmol) de l'acide obtenu au stade H et 770 µl (1,3 éq) de chloroformiate d'éthyle en présence de triéthylamine dans l'acétone conduit à l'anhydride mixte qui est alors traité par 550 mg (1,3 éq) d'azoture de sodium. L'acylazide subit alors un réarrangement dans 40 ml de toluène anhydre à 80°C pour conduire à l'isocyanate. Celui ci est agité 36 heures à température ambiante dans une solution aqueuse d'acide chlorhydrique à 20 % pour conduire à l'amine du titre.

Selon la même procédure, on obtient les Préparations suivantes

### Préparation 2 : Trans 2-(2,7-diméthoxy-1-naphtyl)-1-cyclopropanamine

### Préparation 3 : Trans 2-(2-méthoxy-1-naphtyl)-1-cyclopropanamine

### Préparation 4 : Trans 2-(5-méthoxybenzo[b]furan-3-yl)-1-cyclopropanamine

### Préparation 5 : Trans 2-(5-méthoxybenzo[b]thiophèn-3-yl)-1-cyclopropanamine

### Préparation 6 : Trans 2-(5-méthoxy-1H-pyrrolo[2,3-b]pyridin-3-yl)-1-cyclopropanamine

### Préparation 7 : Trans 2-(5-méthoxy-2-phénylbenzo[b]furan-3-yl)-1-cyclopropanamine

### Préparation 8 : Trans 2-(5-méthoxy-2-benzylbenzo[b]furan-3-yl)-1-cyclopropanamine

### Préparation 9 : Trans 2-(5-éthylbenzo[b]thiophèn-3-yl)-1-cyclopropanamine

### Préparation 10 : Trans 2-(5-méthoxy-2-phénylbenzo[b]thiophèn-3-yl)-1-cyclopropanamine

### Préparation 11 : Trans 2-(5-méthoxy-2-benzylbenzo[b]thiophèn-3-yl)-1-cyclopropanamine

### Préparation 12 : Trans 2-(7-méthoxy-3-phényl-1-naphtyl)-1-cyclopropanamine

### Préparation 13 : Trans 2-{7-méthoxy-3-[3-(trifluorométhyl)phényl]-1-naphtyl}-1-cyclopropanamine

### Préparation 14 : Trans 2-(5-méthoxy-2-phényl-1H-pyrrolo[2,3-b]pyridin-3-yl)-1-cyclopropanamine

### Préparation 15 : Trans 2-(2,3-dihydro-1H-benzo[f]chromèn-10-yl)-1-cyclopropanamine

### Préparation 16 : Trans 2-(8,9-dihydro-7H-furo[3,2-f]chromèn-1-yl)-1-cyclopropanamine

### Préparation 17 : Trans 2-(2-méthoxy-1-naphtyl)-1-cyclopropane carbonitrile

### Stade A : 3-(2-Méthoxy-1-naphtyl)-2-propènenitrile

A partir de 5 g (26,85 mmol) de 2-méthoxynaphtaldéhyde dans 30 ml de THF anhydre en présence de 1,3 g (1,2 eq) de NaH (60 % dans l'huile) dans 15 ml de THF anhydre et de 6,5 ml (1,2 eq) de diéthyl cyanométhylphosphonate on obtient le produit du titre sous forme d'un solide blanc.

### Stade B : Trans 2-(2-méthoxy-1-naphtyl)-1-cyclopropane carbonitrile

5 g (23,9 mmol) du composé obtenu au stade A en solution dans 50 ml de DMSO sont additionnés goutte à goutte sur l'ylure formé à partir de 7,9 g (1,5 eq) d'iodure de triméthylsulfoxonium et de 1,15 g (1,2 eq) de NaH (60 % en suspension dans l'huile) dans 20 ml de DMSO. Le dérivé du titre est obtenu sous forme d'huile et purifié par chromatographie sur gel de silice (éluant : CH₂Cl₂/iPrOH 98/2).

Selon la même procédure, on obtient les Préparations suivantes

### Préparation 18 : Trans 2-(2,7-diméthoxy-1-naphtyl)-1-cyclopropane carbonitrile

### Préparation 19 : Trans 2-(7-méthoxy-1-naphtyl)-1-cyclopropane carbonitrile

### Préparation 20 : Trans 2-(7-méthoxy-3-phényl-1-naphtyl)-1-cyclopropane carbonitrile

### Préparation 21 : Trans 2-(5-méthoxy-2-phénylbenzo[b]furan-3-yl)-1-cyclopropane carbonitrile

### Préparation 22 : Trans 2-(5-éthylbenzo[b]thiophèn-3-yl)-1-cyclopropane carbonitrile

### Préparation 23 : Trans 2-{5-méthory-2-[3-(trifluorométhyl)benzyl]benzo[b]thiophèn-3-yl}-1-cyclopropane carbonitrile

### Préparation 24 : Trans 2-(5-méthoxy-2-phényl-1H-pyrrolo[2,3-b]pyridin-3-yl)-1-cyclopropane carbonitrile

### Préparation 25 : Trans 2-(2,3-dihydro-1H-benzo[f]chromèn-10-yl)-1-cyclopropane carbonitrile

### Préparation 26 : Trans 2-(8,9-dihydro-7H-furo[3,2-f]chromèn-1-yl)-1-cyclopropane carbonitrile

### Préparation 27 : 3-(2-Méthoxy-1-naphtyl)cyclobutanamine

Une solution de 0,1 mol de cyclobuténone dans 50 ml de THF est ajoutée goutte à goutte au réactif de Grignard formé à partir du 1-Bromo-2-méthoxynaphtalène (0,1 mol) et de 2,5 g de magnésium dans 200 ml de THF. Le milieu réactionnel est agité. En fin de réaction, le milieu est quenché avec HCI 3N et agité pendant 1 heure après dilution dans 200 ml d'éther. Après extraction à l'éther et concentration sous vide de la phase organique, la cyclobutanone correspondante est obtenue et remise en solution avec de l'hydroxylamine. Lorsque l'oxime est formée, le milieu est soumis à une hydrogénation sur Nickel de Raney pour conduire à l'amine du titre.

Les Préparations 28 et 29 sont obtenues en suivant le mode opératoire décrit dans la Préparation 27 à partir des substrats appropriés.

### Préparation 28 : 3-(2,7-Diméthoxy-1-naphtyl)cyclohexanamine

### Préparation 29 : 3-(2,7-Diméthoxy-1-naphtyl)cyclopentanamine

### Préparation 30 : 2-[(7-Méthoxy-1-naphtyl)méthyl]cyclopropanamine

On procède comme dans la Préparation 1 stades D-I à partir du 2-(7-méthoxy-1-naphtyl)acétaldéhyde.

### Préparation 31 : 2-[(7-Méthoxy-3-phényl-1-naphtyl)méthyl]cyclopropane carbonitrile

On procède comme dans la Préparation 17 à partir du 2-(7-méthoxy-3-phényl) acétaldéhyde.

### Préparation 32 : Acide 2-(8,9-dihydro-7H-furo[3,2-f]chromèn-1-ylméthyl) cyclopropane carboxylique

### Stade A : 2-(8,9-Dihydro-7H-furo[3,2-f]chromèn-1-ylméthyl)cyclopropane carbonitrile

On procède comme dans la Préparation 17 à partir du 2-(8,9-dihydro-*7H*-furo[3,2-*f*] chromèn-1-yl)acétaldéhyde.

### Stade B : Acide 2 - (8,9 -dihydro -7H-furo[3,2-f]chromèn-1-ylméthyl)cyclopropane carboxylique

On procède à une hydrolyse en milieu acide ou basique du composé obtenu au stade A.

Les Préparations 33 et 34 sont obtenues en procédant comme dans la Préparation 32.

### Préparation 33 : Acide 2-{[5-méthoxy-2-(3-méthoxyphényl)-1-benzofuran-3-yl]méthyl}cyclopentane carboxylique

### Préparation 34 : Acide 2-[(5-Méthoxyfuro[2,3-b]pyridin-3-yl)méthyl]cyclobutane carboxylique

Les Préparations 35 à 38 sont obtenues en procédant comme dans la Préparation 1 en remplaçant le stade G par une cycloaddition avec le substrat approprié.

### Préparation 35 : 2-(5-Ethyl-1-benzothiophèn-3-yl)cyclohexylamine

### Préparation 36 : 2-(5-Ethyl-1-benzothiophèn-3-yl)cyclobutylamine

### Préparation 37 : 2-(8,9-Dihydro-7H-furo[3,2-f]chromèn-1-yl)cyclobutylamine

### Préparation 38 : 2-(5-Méthoxy-1H-pyrrolo[3,2-b]pyridin-3-yl)cyclopentylamine

### EXEMPLE 1 : N-[Trans-2-(7-méthoxy-1-naphtyl)-1-cyclopropyl]acétamide

350 mg du composé obtenu dans la Préparation 1 sont mis en solution dans un mélange CH₂Cl₂/H₂O (5 : 5 ml), en présence de carbonate de sodium. 247µl (1,5éq) d'anhydride acétique sont additionnés goutte à goutte à 0°C. L'amide brut obtenu est purifié par chromatographie sur colonne de gel de silice (éluant : CH₂Cl₂/iPrOH 95 : 5) pour donner un solide blanc qui est recristallisé dans un mélange hexane /CH₂Cl₂.
*Point de fusion :* 136°C

| *Microanalyse élémentaire :* | | | |
|---|---|---|---|
| | %C | %H | %N |
| théorique + 1/5 H₂O | 74,31 | 6,62 | 5,41 |
| expérimentale | 74,32 | 6,69 | 5,44 |

### EXEMPLE 2 : N-[Trans-2-(7-méthoxy-1-naphtyl)-1-cyclopropyl]propanamide

400 mg du composé obtenu dans la Préparation 1 sont mis en solution dans un mélange CH₂Cl₂/H₂O (5 : 5 ml), en présence de carbonate de sodium. 1,5 éq d'anhydride propanoïque sont additionnés goutte à goutte à 0°C. L'amide brut obtenu est purifié par chromatographie sur colonne de gel de silice (éluant : CH₂Cl₂/iPrOH 95 : 5) pour donner un solide blanc qui est recristallisé dans un mélange hexane /CH₂Cl₂.
*Point de fusion :* 125°C

| *Microanalyse élémentaire :* | | | |
|---|---|---|---|
| | %C | %H | %N |
| théorique | 75,55 | 6,92 | 5,34 |
| expérimentale | 75,15 | 7,01 | 5,14 |

### EXEMPLE 3 : N-[Trans-2-(7-méthoxy-1-naphtyl)-1-cyclopropyl]butanamide

150 mg (0,7mmol) du composé obtenu dans la Préparation 1 sont mis en solution dans 10 ml de CH₂Cl₂ anhydre, en présence de 146 µl (1,5 éq) de triéthylamine. A 0°C sous argon, 87 µl (1,2 éq) de chlorure d'acide butyrique sont additionnés goutte à goutte. Après une heure d'agitation, le mélange réactionnel est hydrolysé et l'amide est extrait au CH₂Cl₂. Après chromatographie sur colonne de gel de silice (éluant : CH₂Cl₂/iPrOH 95 : 5), le solide blanc obtenu est recristallisé dans un mélange hexane /CH₂Cl₂.
*Point de fusion :* 119°C

| *Microanalyse élémentaire :* | | | |
|---|---|---|---|
| | %C | %H | %N |
| théorique | 76,32 | 7,42 | 4,94 |
| expérimentale | 76,17 | 7,57 | 4,96 |

### EXEMPLE 4 : N-[Trans-2-(7-méthoxy-1-naphtyl)-1-cyclopropyl]cyclopropane carboxamide

180 mg (0,7 mmol) du composé obtenu dans la Préparation 1 sont mis en solution dans 10 ml de CH₂Cl₂ anhydre, en présence de 141 µl (1,5 éq) de triéthylamine. A 0°C, sous argon 84 µl (1,2 éq) de chlorure de cyclopropane carbonyle sont additionnés goutte à goutte. Après une heure d'agitation, le mélange réactionnel est hydrolysé et l'amide est extrait au CH₂Cl₂. Après chromatographie sur colonne de gel de silice (éluant : CH₂Cl₂/iPrOH 95 : 5), le solide blanc obtenu est recristallisé dans un mélange hexane /CH₂Cl₂.
*Point de fusion :* 165°C

| *Microanalyse élémentaire :* | | | |
|---|---|---|---|
| | %C | %H | %N |
| théorique | 76,84 | 6,80 | 4,98 |
| expérimentale | 76,29 | 6,92 | 4,97 |

### EXEMPLE 5 : N-[Trans-2-(2,7-diméthoxy-1-naphtyl)-1-cyclopropyl]acétamide

400 mg (1,64 mmol) du composé obtenu dans la Préparation 2 sont mis en solution dans un mélange CH₂Cl₂/H₂O (10 :10ml), en présence de carbonate de sodium. 200 µl (1,5 éq) d'anhydride acétique sont additionnés goutte à goutte à 0°C. L'amide brut obtenu est purifié par chromatographie sur silice (éluant : CH₂Cl₂/iPrOH 90 :10) et conduit à un solide blanc qui est recristallisé dans un mélange hexane /CH₂Cl₂.
*Point de fusion :* 128°C

| *Microanalyse élémentaire :* | | | |
|---|---|---|---|
| | %C | %H | %N |
| théorique | 71,49 | 6,66 | 4,90 |
| expérimentale | 70,99 | 6,74 | 4,88 |

### EXEMPLE 6 : N-[Trans-2-(2,7-diméthoxy-1-naphtyl)-1-cyclopropyl]propanamide

500 mg (2 mmol) du composé obtenu dans la Préparation 2 sont mis en solution dans un mélange CH₂Cl₂/H₂O (10 :10 ml), en présence de carbonate de sodium. 215 µl (1,2 éq) d'anhydride propionique sont additionnés goutte à goutte à 0°C. L'amide brut obtenu est purifié par chromatographie sur silice (éluant : CH₂Cl₂/iPrOH 98 : 2) et conduit à un solide blanc qui est recristallisé dans un mélange hexane /CH₂Cl₂.
*Point de fusion :* 135°C

| *Microanalyse élémentaire :* | | | |
|---|---|---|---|
| | %C | %H | %N |
| théorique + 1/3 H₂O | 70,81 | 6,93 | 4,59 |
| expérimentale | 70,86 | 7,00 | 4,58 |

### EXEMPLE 7 : N-[Trans-2-(2,7-diméthoxy-1-naphtyl)-1-cyclopropyl]butanamide

100 mg du composé obtenu dans la Préparation 2 sont mis en solution dans un mélange CH₂Cl₂/H₂O (10 :10 ml), en présence de carbonate de sodium. 43 µl (1,2 éq) d'anhydride butyrique sont additionnés goutte à goutte à 0°C. L'amide brut obtenu est purifié par chromatographie sur silice (éluant : CH₂Cl₂/iPrOH 95 : 5) pour donner une huile qui cristallise lentement à température ambiante et est recristallisé dans un mélange hexane/CH₂Cl₂.

### EXEMPLE 8 : N-[Trans-2-(2-méthoxy-1-naphtyl)-1-cyclopropyl]acétamide

500 mg du composé obtenu dans la Préparation 3 sont mis en solution dans un mélange CH₂Cl₂/H₂O (10 :10 ml), en présence de carbonate de sodium. 332 µl (1,5 éq) d'anhydride acétique sont additionnés goutte à goutte à 0°C. L'amide brut obtenu est purifié par chromatographie sur colonne de gel de silice (éluant : CH₂Cl₂/iPrOH 90 :10) et conduit à une huile qui cristallise lentement sous forme de cristaux blancs recristallisés dans un mélange hexane/CH₂Cl₂.
*Point de fusion :* 155°C

| *Microanalyse élémentaire :* | | | |
|---|---|---|---|
| | %C | %H | %N |
| théorique | 75,29 | 6,66 | 5,49 |
| expérimentale | 75,35 | 6,65 | 5,39 |

### EXEMPLE 9 : N-[Trans-2-(2-méthoxy-1-naphtyl)-1-cyclopropyl]propanamide

450 mg du composé obtenu dans la Préparation 3 sont mis en solution dans un mélange CH₂Cl₂/H₂O (10 :10 ml), en présence de carbonate de sodium. 400 µl (1,5 éq) d'anhydride propionique sont additionnés goutte à goutte à 0°C. L'amide brut obtenu est purifié par chromatographie sur silice (éluant : CH₂Cl₂/iPrOH 95 : 5) et conduit à une huile qui cristallise lentement sous forme de cristaux beige clair recristallisés dans un mélange hexane /CH₂Cl₂.
*Point de fusion :* 144°C

| *Microanalyse élémentaire :* | | | |
|---|---|---|---|
| | %C | %H | %N |
| théorique | 75,55 | 6,92 | 5,34 |
| expérimentale | 75,72 | 6,92 | 5,17 |

### EXEMPLE 10 : N-[Trans-2-(2-méthoxy-1-naphtyl)-1-cyclopropyl]butanamide

400 mg du composé obtenu dans la Préparation 3 sont mis en solution dans un mélange CH₂Cl₂/H₂O (10 :10 ml), en présence de carbonate de sodium. 460 µl (1,5 éq) d'anhydride butyrique sont additionnés goutte à goutte à 0°C. L'amide brut obtenu est purifié par chromatographie sur silice (éluant : CH₂Cl₂/iPrOH 95 : 5) pour donner un solide blanc qui est recristallisé dans un mélange hexane /CH₂Cl₂.
*Point de fusion :* 113°C

| *Microanalyse élémentaire :* | | | |
|---|---|---|---|
| | %C | %H | %N |
| théorique | 76,32 | 7,42 | 4,94 |
| expérimentale | 76,23 | 7,40 | 4,94 |

### EXEMPLE 11 : N-[Trans-2-(2-méthoxy-1-naphtyl)-1-cyclopropylméthyl]acétamide

A 700 mg (3,1 mmol) du composé obtenu dans la Préparation 17 en solution dans 20 ml de THF est ajouté 1,6 ml (5 eq) d'anhydride acétique et une spatule de Nickel de Raney. Après 24 heures à température ambiante sous atmosphère d'hydrogène, le résidu est chromatographié sur colonne de gel de silice (éluant CH₂Cl₂; CH₂Cl₂/iPrOH 95 : 5). L'acétamide attendu est obtenu sous forme d'huile qui cristallise pour donner un solide blanc qui est recristallisé dans un mélange CH₂Cl₂/hexane.
*Point de fusion :* 96°C

### EXEMPLE 12 : N-[Trans-2-(2-méthoxy-1-naphtyl)-1-cyclopropylméthyl] propanamide

A 600 mg (3,1 mmol) du composé obtenu dans la Préparation 17 en solution dans 20ml de THF est ajouté 1 ml (3 eq) d'anhydride propionique et une spatule de Nickel de Raney. Après 5 heures à 50°C sous atmosphère d'hydrogène le résidu est chromatographié sur colonne de gel de silice (éluant CH₂Cl₂ ; CH₂Cl₂/iPrOH 98 : 2). Le composé du titre est obtenu sous forme d'une huile incolore.

| *Microanalyse élémentaire :* | | | |
|---|---|---|---|
| | %C | %H | %N |
| théorique + 1/5 H₂O | 75,34 | 7,38 | 4,88 |
| expérimentale | 75,35 | 7,66 | 4,67 |

### EXEMPLE 13 : N-[Trans-2-(2-méthoxy-1-naphtyl)-1-cyclopropylméthyl] butanamide

A 550 mg (2,5 mmol) du composé obtenu dans la Préparation 17 en solution dans 20 ml de THF est ajouté 1,2 ml (3 eq) d'anhydride butyrique et une spatule de Nickel de Raney. Après 4 heures à 50°C sous atmosphère d'hydrogène le résidu est chromatographié sur colonne de gel de silice (éluant CH₂Cl₂; CH₂Cl₂/iPrOH 98 : 2). Le composé du titre est obtenu sous forme d'une huile incolore.

| *Microanalyse élémentaire :* | | | |
|---|---|---|---|
| | %C | %H | %N |
| théorique + 1/4 H₂O | 75,60 | 7,68 | 4,64 |
| expérimentale | 75,63 | 7,86 | 4,37 |

En procédant comme dans l'Exemple 1 à partir des Préparations appropriées, on obtient les Exemples 14 à 17

### EXEMPLE 14 : N-[Trans -2-(5-méthoxybenzo[b]furan-3-yl)cyclopropyl]acétamide

### Produit de Départ : Préparation 4

### EXEMPLE 15 : N-[Trans-2-(5-méthoxy-2-phénylbenzo[b]furan-3-yl)cyclopropyl] acétamide

### Produit de Départ : Préparation 7

### EXEMPLE 16 : N-[Trans-(7-méthoxy-3-phényl-1-naphtyl)-1-cyclopropyl]acétamide

### Produit de Départ : Préparation 12

### EXEMPLE 17 : N-[Trans-2-(8,9-dihydro-7H-furo[3,2-f]chromèn-1-yl)-1-cyclopropyl]acétamide

### Produit de Départ : Préparation 16

En procédant comme dans l'Exemple 2 à partir des Préparations appropriées, on obtient les Exemples 18 à 20 :

### EXEMPLE 18 : N-[Trans-2-(5-méthoxy-1H-pyrrolo[2,3-b]pyridin-3-yl)-1-cyclopropyl]propanamide

### Produit de Départ : Préparation 6

### EXEMPLE 19 : N-[Trans-2-(5-méthoxybenzo[b]thiophèn-3-yl)-1-cyclopropyl] propanamide

### Produit de Départ : Préparation 9

### EXEMPLE 20 : N-[Trans-2-{7-méthoxy-3-[3-(trifluorométhyl)phényl]-1-naphtyl} cyclopropyl]propanamide

### Produit de Départ : Préparation 13

En procédant comme dans l'Exemple 3 à partir des Préparations appropriées, on obtient les Exemples 21 à 23 :

### EXEMPLE 21 : N-[Trans-2-(5-méthoxybenzo[b]thiophèn-3-yl)cyclopropyl] butanamide

### Produit de Départ : Préparation 5

### EXEMPLE 22 : N-[Trans-2-(5-méthoxy-2-phénylbenzo[b]thiophèn-3-yl) cyclopropyl]butanamide

### Produit de Départ : Préparation 10

### EXEMPLE 23 : N-[Trans-2-(5-méthoxy-2-phényl-1H-pyrrolo[2,3-b]pyridin-3-yl) cyclopropyl]butanamide

### Produit de Départ : Préparation 14

En procédant comme dans l'Exemple 4 à partir des Préparations appropriées, on obtient les Exemples 24 à 26 :

### EXEMPLE 24 : N-[Trans-2-(5-méthoxy-2-phénylbenzo[b]furan-3-yl)cyclopropyl]-1-cyclopropane carboxamide

### Produit de Départ : Préparation 7

### EXEMPLE 25 : N-[Trans-2-(5-méthoxy-2-benzylbenzo[b]thiophèn-3-yl) cyclopropyl]-1-cyclopropane carboxamide

### Produit de Départ : Préparation 11

### EXEMPLE 26 : N-[Trans-2-(2,5-dihydro-1H-benzo[f]chromèn-10-yl)cyclopropyl]-1-cyclopropane carboxamide

### Produit de Départ : Préparation 15

En procédant comme dans l'Exemple 11 à partir des Préparations appropriées, on obtient les Exemples 27 à 30

### EXEMPLE 27 : N-[Trans-2-(2-méthoxy-1-napthyl)-1-cyclopropylméthyl]acétamide

### Produit de Départ : Préparation 17

### EXEMPLE 28 : N-[Trans-2-(5-méthoxy-3-phényl-1-naphtyl)-1-cyclopropylméthyl] acétamide

### Produit de Départ : Préparation 20

### EXEMPLE 29 : N-[Trans-2-{5-méthoxy-2-[3-(trifluorométhyl)benzyl]benzo[b] thiophèn-3-yl}cyclopropylméthyl]acétamide

### Produit de Départ : Préparation 23

### EXEMPLE 30 : N-{Trans-[2-(2,3-dihydro-1H-benzo[f]chromén-10-yl)-1-cyclopropyl]méthyl}acétamide

### Produit de Départ : Préparation 25

En procédant comme dans l'Exemple 12 à partir des Préparations appropriées, on obtient les Exemples 31 à 34 :

### EXEMPLE 31 : N-[Trans-(2,7-diméthoxy-1-naphtyl)-1-cyclopropylméthyl] propanamide

### Produit de Départ : Préparation 18

### EXEMPLE 32 : N-[Trans-2-(5-méthoxy-2-phénylbenzo[b]furan-3-yl] cyclopropylméthyl]propanamide

### Produit de Départ : Préparation 21

### EXEMPLE 33 : N-{Trans-[2-(5-méthoxy-1-méthyl-2-phényl-1H-pyrrolo[2,3-b] pyridin-3-yl)cyclopropyl]méthyl}propanamide

### Produit de Départ : Préparation 24

### EXEMPLE 34 : N-{Trans-[2-(8,9-dihydro-7H-furo[3,2-f]chromèn-1-yl)cyclopropyl] méthyl}propanamide

### Produit de Départ : Préparation 26

En procédant comme dans l'Exemple 13 à partir des Préparations appropriées, on obtient les Exemples 35 et 36 :

### EXEMPLE 35 : N-[Trans-2-(7-méthoxy-1-naphtyl)cyclopropylméthyl]butanamide

### Produit de Départ : Préparation 19

### EXEMPLE 36 : N-[Trans-2-(5-éthyl-benzo[b]thiophèn-3-yl)cyclopropylméthyl] butanamide

### Produit de Départ : Préparation 22

### EXEMPLE 37 : N-[3-(2-Méthoxy-1-naphtyl)cyclobutyl]acétamide

On procède comme dans l'Exemple 1 à partir du composé obtenu dans la Préparation 27.

### EXEMPLE 38 : N-[3-(2,7-Diméthoxy-1-naphtyl)cyclohexyl]propanamide

On procède comme dans l'Exemple 2 à partir du composé obtenu dans la Préparation 28.

### EXEMPLE 39 : N-[3-(2,7-Diméthoxy-1-naphtyl)cyclopentyl]-N'-méthylurée

Le produit du titre est obtenu après condensation de l'isocyanate de méthyle sur le composé obtenu dans la Préparation 29.

### EXEMPLE 40 : N-{2-[(7-Méthoxy-1-naphtyl)méthyl]cyclopropyl}butanamide

On procède comme dans l'Exemple 3 à partir du composé obtenu dans la Préparation 30.

### EXEMPLE 41 : N-({2-[(7-Méthoxy-3-phényl-1-naphtyl)méthyl]cyclopropyl} méthyl)propanamide

On procède comme dans l'Exemple 12 à partir du composé obtenu dans la Préparation 31.

### EXEMPLE 42 : 2-(8,9-Dihydro-7H-furo[3,2-f]chromèn-1-ylméthyl)-N-méthyl cyclopropane carboxamide

Le produit du titre est obtenu en condensant, en présence d'un agent de couplage, la N-méthylamine sur le composé obtenu dans la Préparation 32.

### EXEMPLE 43 : N-Allyl-2-{[5-méthoxy-2-(3-méthoxyphényl)-1-benzofuran-3-yl]méthyl}cyclopentane carboxamide

Le produit du titre est obtenu par condensation, en présence d'un agent de couplage, de la N-allylamine sur le composé obtenu dans la Préparation 33.

### EXEMPLE 44 : N-(Bromoéthyl)-2-[(5-méthoxyfuro[2,3-b]pyridin-3-yl)méthyl] cyclobutane carboxamide

Le produit du titre est obtenu en condensant, en présence d'un agent de couplage, la N-(bromométhyl)amine sur le composé obtenu dans la Préparation 34.

### EXEMPLE 45 : N-{2-[(5-Ethyl-1-benzothiophèn-3-yl)méthyl]cyclohexyl} butanamide

On procède comme dans l'Exemple 2 à partir du composé obtenu dans la Préparation 35.

### EXEMPLE 46 : N-[2-(5-Ethyl-1-benzothiophèn-3-yl)cyclobutyl]cyclopropane carboxamide

On procède comme dans l'Exemple 4 à partir du composé obtenu dans la Préparation 36.

### EXEMPLE 47 : N-[2-(8,9-Dihydro-7H-furo[3,2-f]chromèn-1-yl)cyclobutyl]-2-méthylpropanamide

On procède comme dans l'Exemple 4 en remplaçant le chlorure de cyclopropane carbonyl par le chlorure de l'acide 2-méthylpropanoïque.

### EXEMPLE 48 : N-[2-(5-Méthoxy-1H-pyrrolo[3,2-b]pyridin-3-yl)cyclopentyl] butanamide

On procède comme dans l'Exemple 2 à partir du composé obtenu dans la Préparation 38.

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Etude de liaison aux récepteurs de la mélatonine sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology, 1, pp 1-4, 1989).

### Protocole

I) Les membranes de pars tuberalis de mouton sont préparées et utilisées comme tissu cible dans des expériences de saturation pour déterminer les capacités et affinités de liaison pour la 2-[¹²⁵I]-iodomélatonine.
2) Les membranes de pars tuberalis de mouton sont utilisées comme tissu cible, avec les différents composés à tester, dans des expériences de liaison compétitive par rapport à la mélatonine.
Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### EXEMPLE C : Etude de liaison aux récepteurs mt₁ et MT₂ de la mélatonine

Les expériences de liaison aux récepteurs mt₁ ou MT₂ sont réalisées en utilisant la 2-[¹²⁵I]-mélatonine comme radioligand de référence. La radioactivité retenue est déterminée à l'aide d'un compteur à scintillation liquide Beckman® LS 6000.

Des expériences de liaison compétitive sont ensuite réalisées en triple, avec les différents composés à tester. Une gamme de concentrations différentes est testée pour chaque composé. Les résultats permettent de déterminer les affinités de liaison des composés testés (IC₅₀).

Ainsi, les valeurs d'IC₅₀ trouvées pour les composés de l'invention montrent que la liaison des composés testés est très puissante pour l'un ou l'autre des sous-types de récepteurs mt₁ ou MT₂, ces valeurs se situant dans un intervalle de 0,1 à 10nM.

### EXEMPLE D : Test des quatre plaques

Les produits de l'invention sont administrés par voie oesophagienne à des lots de dix souris. Un lot reçoit du sirop de gomme. Trente minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des dérivés de l'invention.

### EXEMPLE E : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles Long Evans âgés de un mois sont soumis dès leur arrivée au laboratoire à un cycle lumineux de 12h de lumière par 24h (LD 12 : 12). Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats :

Il apparaît clairement que les composés de l'invention permettent d'agir de façon puissante sur le rythme circadien *via* le système mélatoninergique.

### EXEMPLE F : Activité Antiarythmique

### Protocole

### (Ref: LAWSON J.W. et al. J. Pharmacol. Expert. Therap., 1968, 160, pp 22-31)

La substance testée est administrée par voie intrapéritonéale à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE G : Composition pharmaceutique : Comprimés

| 1000 comprimés dosés à 5 mg de N-[*trans*2-(7-méthoxy-1-naphtyl)-1-cyclopropyl]acétamide | |
|---|---|
| (Exemple 1) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composé de formule (I) : 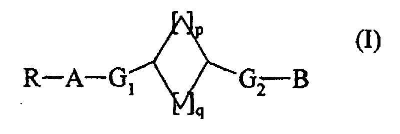 dans laquelle
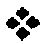 A représente
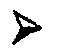 un système cyclique de formule (II) : 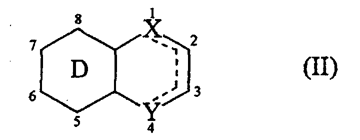 où X et Y, identiques ou différents, représentent un atome d'oxygène, un atome de soufre ou un groupement CH₂,
D représente un cycle benzénique ou un naphtalène,
et la représentation 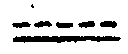 signifie que les liaisons peuvent être simples ou doubles, étant entendu que la valence des atomes est respectée,
dans lequel R substitue le système cyclique D ou le cycle contenant X et Y, et G₁ substitue le cycle contenant X et Y,
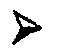 ou un système cyclique de formule (III) : 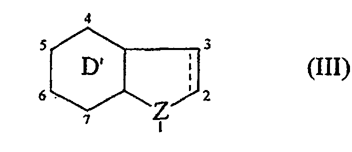 où Z représente un atome d'oxygène, un atome de soufre, un groupement CH₂, ou un groupement NR¹ (dans lequel R¹ représente un atome d'hydrogène, un groupement alkyle (C₁-C₆) linéaire ou ramifié, aryle, ou arylalkyle (C₁-C₆) linéaire ou ramifié),
D' représente un cycle benzénique ou un cycle pyridinique,
et la représentation 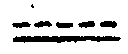 a la même signification que précédemment,
dans lequel R substitue le cycle D' et G₁ substitue l'autre cycle,
étant entendu que les systèmes cycliques de formule (II) ou (III) peuvent être substitués, en plus des groupements R et G₁, par un à trois groupements, identiques ou différents, choisis parmi Rₐ, ORₐ, hydroxyle, CORₐ, formyle, COORₐ, carboxyle ou OCORₐ,
où Rₐ représente un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, polyhalogénoalkyle (C₁-C₆) linéaire ou ramifié, cycloalkyle (C₃-C₈) substitué ou non, cycloalkyle (C₃-C₈) alkyle (C₁-C₆) linéaire ou ramifié substitué ou non, cycloalkényle (C₃-C₈) substitué ou non, cycloalkényle (C₃-C₈) alkyle (C₁-C₆) linéaire ou ramifié substitué ou non, aryle, arylalkyle (C₁-C₆) linéaire ou ramifié, hétéroaryle ou hétéroarylalkyle (C₁-C₆) linéaire ou ramifié,
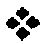 R représente un atome d'hydrogène ou d'halogène, un groupement hydroxyle, SH, Rₐ, ORₐ ou S(O)ₙRₐ où n vaut 0, 1 ou 2 et Rₐ est tel que défini précédemment,
ou forme, avec l'atome de carbone qui le porte et un atome de carbone adjacent un cycle de formule (IV) : 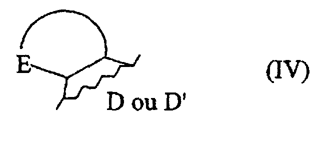 dans laquelle E représente un atome d'oxygène ou un groupement -S(O)ₙ- où n est tel que défini précédemment, le cycle ainsi formé contenant de 5 à 7 atomes, pouvant contenir une ou plusieurs insaturations et pouvant être substitué par un ou plusieurs groupements choisis parmi alkyle (C₁-C₆) linéaire ou ramifié, alkoxy (C₁-C₆) linéaire ou ramifié, carboxy, alkoxycarbonyle (C₁-C₆) linéaire ou ramifié, hydroxy ou oxo,
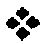 G₁ et G₂, identiques ou différents, représentent une liaison simple ou une chaîne alkylène -(CH₂)ₜ- (dans laquelle t vaut 1, 2, 3 ou 4), substituée ou non par un ou plusieurs radicaux, identiques ou différents, choisis parmi hydroxy, carboxy, formyl, Rₐ, ORₐ, COORₐ, CORₐ, (dans lesquels Rₐ est tel que défini précédemment) ou atomes d'halogène,
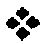 p vaut 0, 1, 2, 3 ou 4,
q vaut 0, 1, 2, 3 ou 4,
avec 1≤p+q≤4,
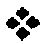 B représente un groupement -NR¹ₐC(Q)R²ₐ, -NR¹ₐC(Q)NR²ₐR³ₐ, ou -C(Q)NR¹ₐR²ₐ dans lesquels R¹ₐ, R²ₐ et R³ₐ, identiques ou différents, peuvent prendre toutes les valeurs de Rₐ et peuvent représenter un atome d'hydrogène, et Q représente un atome d'oxygène ou de soufre,
étant entendu que
- le terme "substitué" affecté aux expressions "alkyle", "alkényle", "alkynyle", signifie que ces groupements sont substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
- le terme "substitué" affecté aux expressions "cycloalkyle", "cycloalkylalkyle", "cycloalkényle", "cycloalkénylalkyle", signifie que la partie cyclique de ces groupements est substituée par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
- par "aryle" on entend les groupements phényle, naphtyle ou biphényle, ces groupements pouvant être substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, cyano, carboxy, alkoxy (C₁-C₆) linéaire ou ramifié carbonyle, nitro, amino ou atomes d'halogène,
- par "hétéroaryle" on entend tout groupement aromatique mono ou polycyclique contenant 5 à 10 atomes contenant 1 à 3 hétéroatomes choisis parmi azote, oxygène ou soufre, ces groupements pouvant être substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, cyano, carboxy, alkoxy (C₁-C₆) linéaire ou ramifié carbonyle, nitro, amino ou atomes d'halogène,
étant entendu que :
- lorsque A représente un noyau naphtalène non substitué ou substitué par un groupement méthoxy ou par un groupement méthyle, G₁ et G₂ représentent simultanément une simple liaison, et B représente un groupement 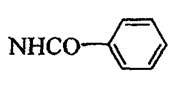 ou NHCOMe, alors on ne peut avoir (q = 0 et p = 4) ou (q = 4 et p = 0),
- lorsque G₁ représente une simple liaison et p+q=1, alors A ne peut représenter un noyau naphtalène substitué par un ou plusieurs atomes d'halogène,
- lorsque A représente un noyau indolique substitué en position 2 par le groupement 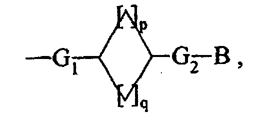 alors B ne peut représenter un groupement urée,
- lorsque A représente un noyau indolique substitué en position 2 par un groupement carboxyle ou alkoxycarbonyle, p vaut 1 et q vaut 0 (ou q vaut 1 et p vaut 0), et G₁ représente une liaison simple, alors B ne peut représenter un groupement CONHAr dans lequel Ar représente un groupement aryle,
leurs énantiomères et diastéréoisomères, ainsi que leurs sels d'addition à un acide ou à une base pharmaceutiquement acceptable.

2. Composés de formule (I) selon la revendication 1 pour lesquels p vaut 1, 2, 3 ou 4 et q vaut 0 (ou p vaut 0 et q vaut 1,2,3 ou 4), leurs énantiomères et diastéréoisomères, ainsi que leurs sels d'addition à un acide ou à une base pharmaceutiquement acceptable.

3. Composés de formule (I) selon la revendication 1 pour lesquels p vaut 1 et q vaut 0 (ou p vaut 0 et q vaut 1), leurs énantiomères et diastéréoisomères, ainsi que leurs sels d'addition à un acide ou à une base pharmaceutiquement acceptable.

4. Composés de formule (I) selon la revendication 3 **caractérisés en ce que** les groupements R-A-G₁- et -G₂-B sont dans une configuration *trans* l'un par rapport à l'autre, leurs énantiomères et diastéréoisomères, ainsi que leurs sels d'addition à un acide ou à une base pharmaceutiquement acceptable.

5. Composés de formule (I) selon la revendication 1 pour lesquels R représente un groupement ORₐ, SRₐ, ou Rₐ, leurs énantiomères et diastéréoisomères, ainsi que leurs sels d'addition à un acide ou à une base pharmaceutiquement acceptable.

6. Composés de formule (I) selon la revendication 1 pour lesquels R représente un groupement ORₐ, leurs énantiomères et diastéréoisomères, ainsi que leurs sels d'addition à un acide ou à une base pharmaceutiquement acceptable.

7. Composés de formule (I) selon la revendication 1 pour lesquels R forme, avec l'atome de carbone qui le porte et un atome de carbone adjacent un cycle de formule (IV), leurs énantiomères et diastéréoisomères, ainsi que leurs sels d'addition à un acide ou à une base pharmaceutiquement acceptable.

8. Composés de formule (I) selon la revendication 1 pour lesquels A représente un système cyclique de formule (II) substitué en position 7 par R et en 1 ou 2 par G₁, A étant non substitué ou substitué (en plus des substituants R et G₁) en position 2 ou 3, leurs énantiomères et diastéréoisomères, ainsi que leurs sels d'addition à un acide ou à une base pharmaceutiquement acceptable.

9. Composés de formule (I) selon la revendication 1 pour lesquels A représente un système cyclique de formule (III) substitué en position 5 par R et en 3 par G₁, A étant non substitué ou substitué (en plus des substituants R et G₁) en position 2, leurs énantiomères et diastéréoisomères, ainsi que leurs sels d'addition à un acide ou à une base pharmaceutiquement acceptable.

10. Composés de formule (I) selon la revendication 1 pour lesquels G₁ représente une liaison simple et G₂ représente une liaison simple ou un groupement CH₂, leurs énantiomères et diastéréoisomères, ainsi que leurs sels d'addition à un acide ou à une base pharmaceutiquement acceptable.

11. Composés de formule (I) selon la revendication 1 pour lesquels B représente un groupement -NHCORₐ ou -CONHRₐ, leurs énantiomères et diastéréoisomères, ainsi que leurs sels d'addition à un acide ou à une base pharmaceutiquement acceptable.

12. Composés de formule (I) selon la revendication 1 pour lesquels A est substitué :
- par un groupement de formule (V) : 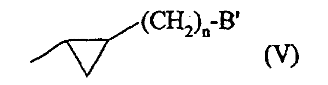 dans laquelle n vaut 0 ou 1 et B' représente un groupement -NHCORₐ ou -CONHRₐ,
- par un groupement ORₐ, SRₐ ou Rₐ,
- et optionnellement par un groupement alkoxy, aryle ou arylalkyle,
leurs énantiomères et diastéréoisomères, ainsi que leurs sels d'addition à un acide ou à une base pharmaceutiquement acceptable.

13. Composés de formule (I) selon la revendication 1 pour lesquels A représente un naphtalène, un chromane ou un benzochromane,
- substitué en 1 ou 2 (formule II) par un groupement de formule (V) : 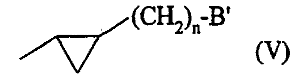
dans laquelle n vaut 0 ou 1 et B' représente un groupement -NHCORₐ ou -CONHRₐ,
- substitué en 7 (formule II) par un groupement ORₐ, SRₐ ou Rₐ,
- et optionnellement substitué en 3 (formule II) par un groupement aryle ou arylalkyle, ou en 2 (formule II) par un groupement alkoxy,
leurs énantiomères et diastéréoisomères, ainsi que leurs sels d'addition à un acide ou à une base pharmaceutiquement acceptable.

14. Composés de formule (I) selon la revendication 1 pour lesquels A représente un benzothiophène, un benzofurane, un indole ou un azaindole,
- substitué en 3 (formule III) par un groupement de formule (V) : 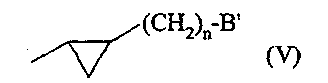
dans laquelle n vaut 0 ou 1 et B' représente un groupement -NHCORₐ ou -CONHRₐ,
- substitué en 5 (formule III) par un groupement ORₐ, SRₐ ou Rₐ,
- et optionnellement substitué en 2 (formule III) par un groupement aryle ou arylalkyle,
leurs énantiomères et diastéréoisomères, ainsi que leurs sels d'addition à un acide ou à une base pharmaceutiquement acceptable.

15. Composés de formule (I) selon la revendication 1 pour lesquels A représente un naphtalène,
- substitué en 1 par un groupement de formule (V) : 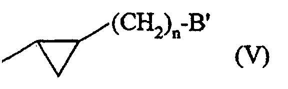
dans laquelle n vaut 0 ou 1 et B' représente un groupement -NHCORₐ ou -CONHRₐ,
- substitué en 7 par un groupement ORₐ,
- et optionnellement substitué en 3 par un groupement aryle ou arylalkyle, ou en 2 par un groupement ORₐ,
leurs énantiomères et diastéréoisomères, ainsi que leurs sels d'addition à un acide ou à une base pharmaceutiquement acceptable.

16. Composés de formule (I) selon la revendication 1 qui sont :
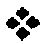 N-[2-(7-méthoxy-1-naphtyl)-1-cyclopropyl]acétamide
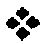 N-[2-(7-méthoxy-1-naphtyl)-1-cyclopropyl]propanamide
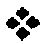 N-[2-(7-méthoxy-1-naphtyl)-1-cyclopropyl]butanamide
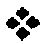 N-[2-(7-méthoxy-1-naphtyl)-1-cyclopropyl]cyclopropane carboxamide
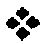 N-[2-(2,7-diméthoxy-1-naphtyl)-1-cyclopropyl]acétamide
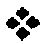 N-[2-(2,7-diméthoxy-1-naphtyl)-1-cyclopropyl]propanamide
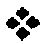 N-[2-(2,7-diméthoxy-1-naphtyl)-1-cyclopropyl]butanamide
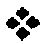 N-[2-(2-méthoxy-1-naphtyl)-1-cyclopropyl]acétamide
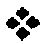 N-[2-(2-méthoxy-1-naphtyl)-1-cyclopropyl]propanamide
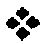 N-[2-(2-méthoxy-1-naphtyl)-1-cyclopropyl]butanamide
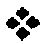 N-[2-(2-méthoxy-1-naphtyl)-1-cyclopropylméthyl]acétamide
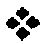 N-[2-(2-méthoxy-1-naphtyl)-1-cyclopropylméthyl]propanamide
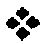 N-[2-(2-méthoxy-1-naphtyl)-1-cyclopropylméthyl]butanamide
leurs énantiomères et diastéréoisomères, ainsi que leurs sels d'addition à un acide ou à une base pharmaceutiquement acceptable.

17. Composés de formule (I) selon la revendication 15 **caractérisés en ce que** les groupements -(CH₂)ₙ-B' et R-A-G₁- sont dans une configuration *trans* l'un par rapport à l'autre, leurs énantiomères et diastéréoisomères, ainsi que leurs sels d'addition à un acide ou à une base pharmaceutiquement acceptable.

18. Procédé de préparation des composés de formule (I) selon la revendication 1 **caractérisé en ce que** l'on utilise comme produit de départ le composé de formule (VI) 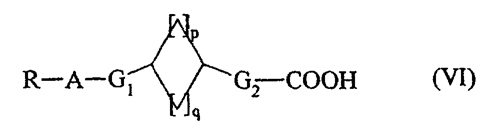 dans laquelle R, A, G₁, G₂, p et q sont tels que définis précédemment,
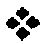 qui est soumis, après activation sous forme de chlorure d'acide ou en présence d'agent de couplage, à l'action d'une amine HNR¹ₐR²ₐ, dans laquelle R¹ₐ et R²ₐ sont tels que définis précédemment pour conduire au composé de formule (I/a), cas particulier des composés de formule (I) : 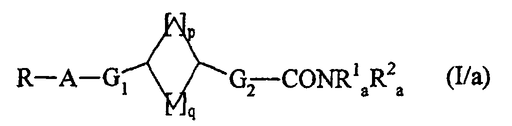 dans laquelle R, A, G₁, G₂, R¹ₐ, R²ₐ, p et q sont tels que définis précédemment,
qui peut être soumis à un agent de thionation pour obtenir le composé de formule (I/b), cas particulier des composés de formule (I), 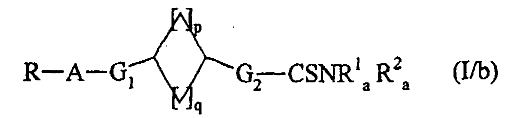 dans laquelle R, A, G₁, G₂, R¹ₐ, R²ₐ, p et q sont définis comme précédemment,
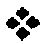 ou soumis, après transformation en azidure correspondant à un réarrangement de Curtius pour conduire après hydrolyse à l'amine de formule (VII) : 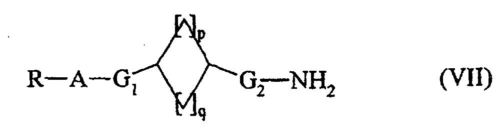 dans laquelle R, A, G₁, G₂, p et q sont tels que définis précédemment,
qui est :
- soit mise en réaction avec un chlorure d'acyle ClCOR¹ₐ ou l'anhydride mixte ou symétrique correspondant pour lesquels R¹ₐ est défini comme précédemment pour conduire au composé de formule (I/c), cas particulier des composés de formule (I) : 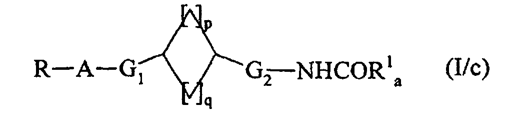 dans laquelle R, A, G₁, G₂, R¹ₐ, p et q sont définis comme précédemment, suivi éventuellement de l'action d'un composé de formule (VIII) :
Rₐ―J (VIII)
dans laquelle Rₐ est tel que défini précédemment et J représente un groupe partant comme un atome d'halogène ou un groupement tosyle,
pour conduire au composé de formule (I/d), cas particulier des composés de formule (I) : 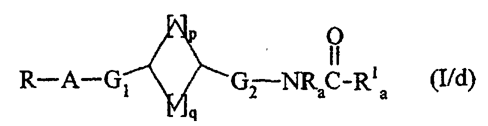 dans laquelle R, A, G₁, G₂, Rₐ, R¹ₐ, p et q sont tels que définis précédemment,
l'ensemble des composés (I/c) et (I/d) formant le composé de formule (I/e), cas particulier des composés de formule (I) 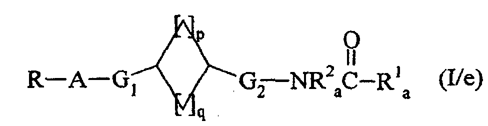 dans laquelle R, A, G₁, G₂, R¹ₐ, R²ₐ, p et q sont tels que définis précédemment,
composé de formule (I/e) qui peut être soumis à un agent de thionation pour conduire au composé de formule (I/f), cas particulier des composés de formule (I) : 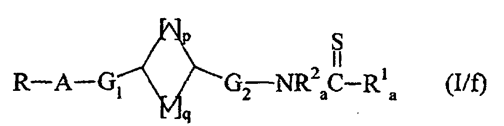 dans laquelle R, A, G₁, G₂, R¹ₐ, R²ₐ, p et q sont tels que définis précédemment,
- soit soumise à l'action d'un composé de formule (IX) :
Q=C=N-R¹ₐ (IX)
dans laquelle Q et R¹ₐ sont tels que définis précédemment,
suivi éventuellement de l'action d'un composé de formule (VIII) pour conduire au composé de formule (I/g), cas particulier des composés de formule (I) : 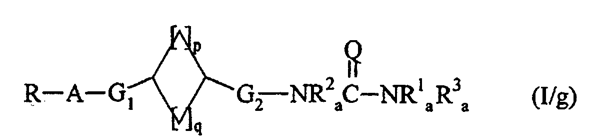 dans laquelle R, A, G₁, G₂, R¹ₐ, R²ₐ, R³ₐ, Q, p et q sont définis comme précédemment,
les composés (I/a) à (I/g) formant l'ensemble des composés de formule (I) et pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

19. Compositions pharmaceutiques contenant les produits de formule (I) selon l'une quelconque des revendications 1 à 17 ou un de leurs sels d'addition avec un acide ou une base pharmaceutiquement acceptable en combinaison avec un ou plusieurs excipients pharmaceutiquement acceptables.

20. Compositions pharmaceutiques selon la revendication 19 utiles pour la fabrication de médicaments pour traiter les troubles du système mélatoninergique.

## Patentansprüche

1. Verbindung der Formel (I): 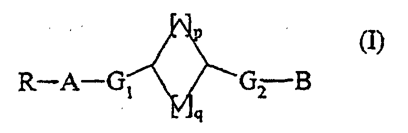 in der
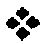 A:
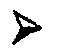 ein cyclisches System der Formel (II): 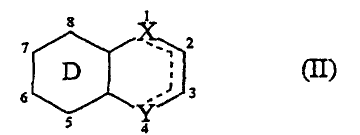
worin X und Y, die gleichartig oder verschieden sind, ein Sauerstoffatom, ein Schwefelatom oder eine Gruppe CH₂ darstellen.
D einen Benzol- oder Naphthalin-Ring bedeutet und
das Symbol 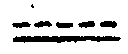 bedeutet, daß die Bindungen einfach oder doppelt sein können, wobei es sich versteht, daß die Wertigkeit der Atome respektiert wird,
worin R das cyclische System D oder den X und Y enthaltenden Ring substituiert und G₁ den X und Y enthaltenden Ring substituiert,
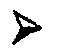 oder ein cyclisches System der Formel (III): 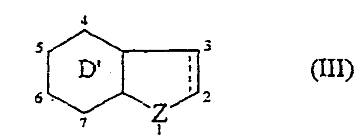 worin Z ein Sauerstoffatom, ein Schwefelatom, eine Gruppe CH₂ oder eine Gruppe NR¹ (worin R¹ ein Wasserstoffatom, eine geradkettige oder verzweigte (C₁-C₆)-Alkylgruppe, Arylgruppe oder geradkettige oder verzweigte Aryl-(C₁-C₆)-alkylgruppe darstellt) bedeutet,
D' einen Benzol- oder Pyridin-Ring bedeutet und
das Symbol 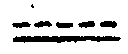 die oben angegebenen Bedeutungen besitzt,
worin R den Ring D' substituiert und G₁ den anderen Ring substituiert, mit der Maßgabe, daß die cyclischen Systeme der Formeln (II) und (III) zusätzlich zu den Gruppen R und G₁ durch ein bis drei gleichartige oder verschiedene Gruppen ausgewählt aus Rₐ, ORₐ, Hydroxyl, CORₐ, Formyl, COORₐ, Carboxyl oder OCORₐ substituiert sein können,
worin Rₐ eine geradkettige oder verzweigte, gegebenenfalls substituierte (C₁-C₆)-Alkylgruppe, geradkettige oder verzweigte, gegebenenfalls substituierte (C₂-C₆)-Alkenylgruppe, geradkettige oder verzweigte. gegebenenfalls substituierte (C₂-C₆)-Alkinylgruppe. geradkettige oder verzweigte Polyhalogenalkylgruppe, gegebenenfalls substituierte (C₃-C₈)-Cycloalkylgruppe, geradkettige oder verzweigte, gegebenenfalls substituierte (C₃-C₈)-Cycloalkyl-(C₁-C₆)-alkylgruppe, gegebenenfalls substituierte (C₃-C₈)-Cycloalkenylgruppe, geradkettige oder verzweigte, gegebenenfalls substituierte (C₃-C₈)-Cycloalkenyl-(C₁-C₆)-alkylgruppe, Arylgruppe, geradkettige oder verzweigte Aryl-(C₁-C₆)-alkylgruppe. Heteroarylgruppe oder geradkettige oder verzweigte Heteroaryl-(C₁-C₆)-alkylgruppe bedeutet, darstellt,
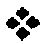 R ein Wasserstoffatom, ein Halogenatom, eine Hydroxylgruppe oder eine Gruppe SH, Rₐ, ORₐ oder S(O)ₙRₐ darstellt, worin n 0, 1 oder 2 bedeutet und Rₐ die oben angegebenen Bedeutungen besitzt,
oder zusammen mit dem ihn tragenden Kohlenstoffatom und einem benachbarten Kohlenstoffatom einen Ring der Formel (IV) bildet: 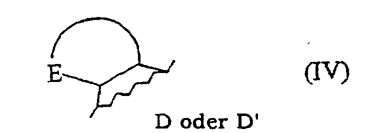 in der E ein Sauerstoffatom oder eine Gruppe -S(O)ₙ- darstellt, worin n die oben angegebenen Bedeutungen besitzt,
wobei der in dieser Weise gebildete Ring 5 bis 7 Atome enthält, eine oder mehrere Unsättigungen aufweisen kann und durch eine oder mehrere Gruppen ausgewählt aus geradkettigem oder verzweigtem (C₁-C₆)-Alkyl, geradkettigem oder verzweigtem (C₁-C₆)-Alkoxy, Carboxy, geradkettigem oder verzweigtem (C₁-C₆)-Alkoxycarbonyl. Hydroxy oder Oxo substituiert sein kann,
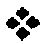 G₁ und G₂, die gleichartig oder verschieden sind, eine Einfachbindung oder eine Alkylenkette -(CH₂)ₜ- (in der t 1, 2, 3 oder 4 bedeutet), die gegebenenfalls durch eine oder mehrere gleichartige oder verschiedenartige Substituenten ausgewählt aus Hydroxy, Carboxy, Formyl, Rₐ, ORₐ, COORₐ, CORₐ (worin Rₐ die oben angegebenen Bedeutungen besitzt) oder Halogenatomen substituiert ist, bedeuten,
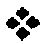 p 0, 1, 2, 3 oder 4 bedeutet,
q 0, 1, 2, 3 oder 4 bedeutet,
mit der Maßgabe, daß die Beziehung 1 ≤ p + q ≤ 4 erfüllt ist,
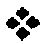 B eine Gruppe -NR¹ₐC(Q)R²ₐ, -NR¹ₐC(Q)NR²ₐR³ₐ oder -C(Q)NR¹ₐR²ₐ bedeutet, worin R¹ₐ, R²ₐ und R³ₐ, die gleichartig oder verschieden sind, sämtliche Bedeutungen von Rₐ annehmen können und ein Wasserstoffatom bedeuten können und Q ein Sauerstoff- oder Schwefelatom bedeutet,
mit der Maßgabe, daß:
- der Begriff "substituiert", bezogen auf die Begriffe "Alkyl", "Alkenyl", "Alkinyl" bedeutet, daß diese Gruppen durch eine oder mehrere, gleichartige oder verschiedene Gruppen ausgewählt aus Hydroxy, geradkettigem der verzweigtem (C₁-C₆)-Alkoxy, geradkettigem oder verzweigtem (C₁-C₆)-Alkyl, geradkettigem oder verzweigtem (C₁-C₆)-Polyhalogenalkyl, Amino oder Halogenatomen substituiert sind,
- der Begriff "substituiert", bezogen auf die Begriffe "Cycloalkyl", "Cycloalkylalkyl", "Cycloalkenyl" oder "Cycloalkenylalkyl" bedeutet, daß der cyclische Teil dieser Gruppen durch eine oder mehrere gleichartige oder verschiedene Reste ausgewählt aus Hydroxy, geradkettigem oder verzweigtem (C₁-C₆)-Alkoxy, geradkettigem oder verzweigtem (C₁-C₆)-Alkyl, geradkettigem oder verzweigtem (C₁-C₆)-Polyhalogenalkyl, Amino oder Halogenatomen substituiert sind,
- unter "Aryl" Phenyl-, Naphthyl- oder Biphenylgruppen zu verstehen sind, wobei diese Gruppen durch eine oder mehrere gleichartige oder verschiedene Reste ausgewählt aus Hydroxy, geradkettigem oder verzweigtem (C₁-C₆)-Alkoxy, geradkettigem oder verzweigtem (C₁-C₆)-Alkyl, geradkettigem oder verzweigtem (C₁-C₆)-Polyhalogenalkyl, Cyano, Carboxy, geradkettigem oder verzweigtem (C₁-C₆)-Alkoxycarbonyl, Nitro, Amino oder Halogenatomen substituiert sein können,
- unter "Heteroaryl" jede aromatische, mono- oder polycyclische Gruppe zu verstehen ist, die 5 bis 10 Atome enthält und 1 bis 3 Heteroatome ausgewählt aus Stickstoff, Sauerstoff oder Schwefel enthält, wobei diese Gruppen durch eine oder mehrere, gleiche oder verschiedene Reste ausgewählt aus Hydroxy, geradkettigem oder verzweigtem (C₁-C₆)-Alkoxy, geradkettigem oder verzweigtem (C₁-C₆)-Alkyl, geradkettigem oder verzweigtem (C₁-C₆)-Polyhalogenalkyl, Cyano, Carboxy, geradkettigem oder verzweigtem (C₁-C₆)-Alkoxycarbonyl, Nitro, Amino oder Halogenatomen substituiert sein können,
wobei es sich versteht, daß:
- wenn A einen nichtsubstituierten oder durch eine Methoxygruppe oder eine Methylgruppe substituierten Phenylkern darstellt, G₁ und G₂ gleichzeitig eine Einfachbindung und B eine Gruppe 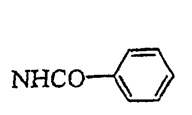 oder NHCOMe bedeuten, dann die Beziehungen (q = 0 und p = 4) oder (q = 4 und p = 0) nicht erfüllt sind,
- wenn G₁ eine Einfachbindung und p + q = 1 ist, dann A nicht einen durch ein oder mehrere Halogenatome substituierten Naphthalinkern bedeutet,
- wenn A einen in der 2-Stellung durch die Gruppe 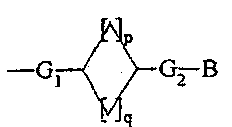 substituierten Indolkern darstellt, B keine Harnstoffgruppe darstellt,
- wenn A einen in der 2-Stellung durch eine Carboxylgruppe oder eine Alkoxycarbonylgruppe substituierten Indolkern darstellt, p gleich 1 und q gleich 0 bedeutet (oder q gleich 1 und p gleich 0 bedeutet), und G₁ eine Einfachbindung darstellt, B nicht eine Gruppe CONHAr darstellt, in der ar eine Arylgruppe bedeutet,
deren Enantiomere und Diastereoisomere sowie deren Additionssalze mit einer pharmazeutisch annehmbaren Säure oder Base.

2. Verbindungen der Formel (I) nach Anspruch 1, worin p gleich 1, 2, 3 oder 4 und q gleich 0 (oder p gleich 0 und q gleich 1, 2, 3 oder 4) bedeuten, deren Enantiomere und Diastereoisomere sowie deren Additionssalze mit einer pharmazeutisch annehmbaren Säure oder Base.

3. Verbindungen der Formel (I) nach Anspruch 1, worin p gleich 1 und q gleich 0 (oder p gleich 0 und q gleich 1) bedeuten, deren Enantiomere und Diastereoisomere sowie deren Additionssalze mit einer pharmazeutisch annehmbaren Säure oder Base.

4. Verbindungen der Formel (I) nach Anspruch 3, **dadurch gekennzeichnet, daß** die Gruppen R-A-G₁ und -G2-B in der *trans*-Konfiguration zueinander stehen, deren Enantiomere und Diastereoisomere sowie deren Additionssalze mit einer pharmazeutisch annehmbaren Säure oder Base.

5. Verbindungen der Formel (I) nach Anspruch 1, worin R eine Gruppe ORₐ, SRₐ oder Rₐ bedeutet, deren Enantiomere und Diastereoisomere sowie deren Additionssalze mit einer pharmazeutisch annehmbaren Säure oder Base.

6. Verbindungen der Formel (I) nach Anspruch 1, worin R eine Gruppe ORₐ darstellt, deren Enantiomere und Diastereoisomere sowie deren Additionssalze mit einer pharmazeutisch annehmbaren Säure oder Base.

7. Verbindungen der Formel (I) nach Anspruch 1, worin R zusammen mit dem ihn tragenden Kohlenstoffatom und einem benachbarten Kohlenstoffatom einen Ring der Formel (IV) bildet, deren Enantiomere und Diastereoisomere sowie deren Additionssalze mit einer pharmazeutisch annehmbaren Säure oder Base.

8. Verbindungen der Formel (I) nach Anspruch 1, worin A ein cyclisches System der Formel (II) darstellt, welches in der 7-Stellung durch R und in der 1-oder 2-Stellung durch G₁ substituiert ist, wobei A nicht substituiert ist oder (neben den Substituenten R und G₁) in der 2- oder 3-Stellung substituiert ist, deren Enantiomere und Diastereoisomere sowie deren Additionssalze mit einer pharmazeutisch annehmbaren Säure oder Base.

9. Verbindungen der Formel (I) nach Anspruch 1, worin A ein cyclisches System der Formel (III) darstellt, welches in der 5-Stellung durch R und in der 3-Stellung durch G₁ substituiert ist, wobei A nicht substituiert ist oder (neben den Substituenten R und G₁) in der 2-Stellung substituiert ist, deren Enantiomere und Diastereoisomere sowie deren Additionssalze mit einer pharmazeutisch annehmbaren Säure oder Base.

10. Verbindungen der Formel (I) nach Anspruch 1, worin G₁ eine Einfachbindung und G₂ eine Einfachbindung oder eine Gruppe CH2 bedeuten, deren Enantiomere und Diastereoisomere sowie deren Additionssalze mit einer pharmazeutisch annehmbaren Säure oder Base.

11. Verbindungen der Formel (I) nach Anspruch 1, worin B eine Gruppe -NHCORₐ oder -CONHRₐ bedeutet, deren Enantiomere und Diastereoisomere sowie deren Additionssalze mit einer pharmazeutisch annehmbaren Säure oder Base.

12. Verbindungen der Formel (I) nach Anspruch 1, worin A substituiert ist:
- durch eine Gruppe der Formel (V): 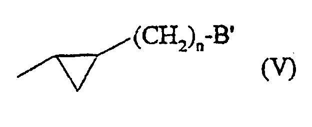 in der n gleich 0 oder 1 und B' eine Gruppe -NHCORₐ oder -CONHRₐ bedeutet,
- durch eine Gruppe ORₐ, SRₐ oder Rₐ,
- und gegebenenfalls durch eine Alkoxy-, Aryl- oder Arylalkylgruppe,
deren Enantiomere und Diastereoisomere sowie deren Additionssalze mit einer pharmazeutisch annehmbaren Säure oder Base.

13. Verbindungen der Formel (I) nach Anspruch 1, worin A ein Naphthalin-, Chroman oder Benzochroman-System bedeutet,
- welches in der 1- oder 2-Stellung (Formel II) durch eine Gruppe der Formel (V) substituiert ist: 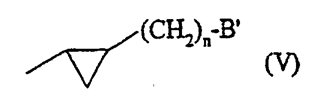 in der n den Wert 0 oder 1 besitzt und B' eine Gruppe -NHCORₐ oder eine Gruppe -CONHRₐ darstellt,
- in der 7-Stellung (Formel II) durch eine Gruppe ORₐ, SRₐ oder Rₐ substituiert ist,
- und gegebenenfalls in der 3-Stellung (Formel II) durch eine Aryl- oder Arylalkylgruppe oder in der 2-Stellung (Formel II) durch eine Alkoxygruppe substituiert ist.
deren Enantiomere und Diastereoisomere sowie deren Additionssalze mit einer pharmazeutisch annehmbaren Säure oder Base.

14. Verbindungen der Formel (I) nach Anspruch 1, worin A ein Benzothiophen-, Benzofuran-, Indol- oder Azaindol-System bedeutet,
- welches in der 3-Stellung (Formel III) durch eine Gruppe der Formel (V) substituiert ist: 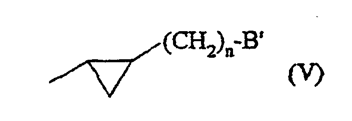 in der n den Wert 0 oder 1 besitzt und B' eine Gruppe -NHCORₐ oder eine Gruppe -CONHRₐ darstellt.
- in der 5-Stellung (Formel III) durch eine Gruppe ORₐ, SRₐ oder Rₐ substituiert ist,
- und gegebenenfalls in der 2-Stellung (Formel III) durch eine Aryl- oder Arylalkylgruppe substituiert ist,
deren Enantiomere und Diastereoisomere sowie deren Additionssalze mit einer pharmazeutisch annehmbaren Säure oder Base.

15. Verbindungen der Formel (I) nach Anspruch 1, worin A ein Naphthalin-System bedeutet,
- welches in der 1-Stellung durch eine Gruppe der Formel (V) substituiert ist: 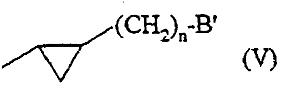 in der n 0 oder 1 darstellt und B' eine Gruppe -NHCORₐ oder eine Gruppe -CONHRₐ bedeutet,
- welches in der 7-Stellung durch eine Gruppe ORₐ substituiert ist,
- und gegebenenfalls in der 3-Stellung durch eine Aryl- oder Arylalkylgruppe oder in der 2-Stellung durch eine Gruppe ORₐ substituiert ist,
deren Enantiomere und Diastereoisomere sowie deren Additionssalze mit einer pharmazeutisch annehmbaren Säure oder Base.

16. Verbindungen der Formel (I) nach Anspruch 1, nämlich:
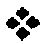 N-[2-(7-methoxy- 1 -naphthyl)-1-cyclopropyll-acetamid,
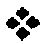 N-[2-(7-methoxy-1-naphthyl)-1-cyclopropyl]-propanamid,
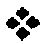 N-[2-(7-methoxy-1-naphthyl)-1-cyclopropyl]-butanamid,
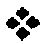 N-[2-(7-methoxy-1-naphthyl)-1-cyclopropyl]-cyclopropan-carboxamid,
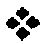 N-[2-(2,7-dimethoxy-1-naphthyl]-1-cyclopropyl]-acetamid,
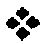 N-[2-(2,7-dimethoxy-1-naphthyl)-1-cyclopropyl]-propanamid,
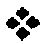 N-[2-(2,7-dimethoxy-1-naphthyl)-1-cyclopropyl]-butanamid,
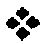 N-[2-(2-methoxy-1-naphthyl)-1-cyclopropyl]-acetamid,
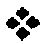 N-[2-(2-methoxy-1-naphthyl)-1-cyclopropyl]-propanamid,
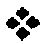 N-[2-(2-methoxy-1-naphthyl)-1-cyclopropyl]-butanamid,
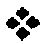 N-[2-(2-methoxy-1-naphthyl)-1-cyclopropylmethyl]-acetamid,
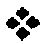 N-[2-(2-methoxy-1-naphthyl)-1-cyclopropylmethyl]-propanamid,
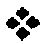 N-[2-(2-methoxy-1-naphthyl)-1-cyclopropylmethyl]-butanamid,
deren Enantiomere und Diastereoisomere sowie deren Additionssalze mit einer pharmazeutisch annehmbaren Säure oder Base.

17. Verbindungen der Formel (I) nach Anspruch 15, **dadurch gekennzeichnet, daß** die Gruppen -(CH₂)ₙ-B' und R-A-G₁- in der *trans*-Stellung zueinander stehen, deren Enantiomere und Diastereoisomere sowie deren Additionssalze mit einer pharmazeutisch annehmbaren Säure oder Base.

18. Verfahren zur Herstellung der Verbindungen der Formel (I) nach Anspruch 1, **dadurch gekennzeichnet, daß** man als Ausgangsprodukt die Verbindung der Formel (VI) verwendet: 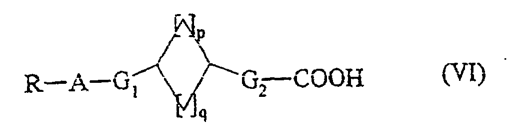 in der R, A, G₁, G₂, p und q die oben angegebenen Bedeutungen besitzen,
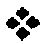 welche man nach der Aktivierung in Form des Säurechlorids oder in Gegenwart eines Kupplungsmittels mit einem Amin HNR¹ₐR²ₐ, worin R¹ₐ und R²ₐ die oben angegebenen Bedeutungen besitzen, kuppelt zur Bildung der Verbindung der Formel (I/a), einem Sonderfall der Verbindungen der Formel (I): 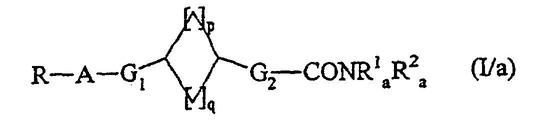 in der R, A. G₁. G₂, R¹ₐ, R²ₐ, p und q die oben angegebenen Bedeutungen besitzen,
welche man der Einwirkung eines Thionierungsmittels unterwerfen kann zur Bildung der Verbindung der Formel (I/b), einem Sonderfall der Verbindungen der Formel (I): 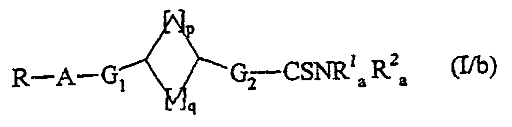 in der R, A, G₁, G₂, R¹ₐ, R²ₐ, p und q die oben angegebenen Bedeutungen besitzen,
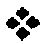 oder nach der Umwandlung in das entsprechende Azid einer Curtius-Umlagerung unterwirft, so daß man nach der Hydrolyse das Amin der Formel (VII) erhält: 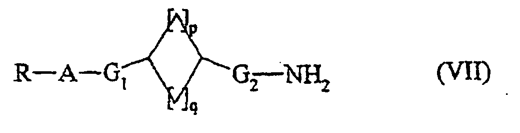 in der R, A, G₁, G₂, p und q die oben angegebenen Bedeutungen besitzen, welches:
- entweder mit einem Acylchlorid ClCOR¹ₐ oder dem entsprechenden gemischten oder symmetrischen Anhydrid, worin R¹ₐ die oben angegebenen Bedeutungen besitzt, umgesetzt wird zur Bildung der Verbindung der Formel (I/c), einem Sonderfall der Verbindungen der Formel (I): 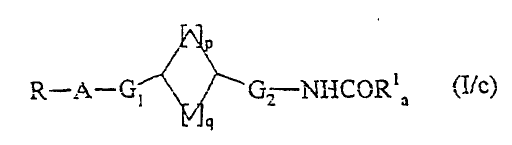 in der R, A, G₁, G₂, R¹ₐ, p und q die oben angegebenen Bedeutungen besitzen.
gegebenenfalls gefolgt von der Einwirkung einer Verbindung der Formel (VIII):
Rₐ - J (VIII)
in der Rₐ die oben angegebenen Bedeutungen besitzt und J eine austretende Gruppe, wie ein Halogenatom oder eine Tosylgruppe darstellt,
zur Bildung der Verbindung der Formel (I/d), einem Sonderfall der Verbindungen der Formel (I): 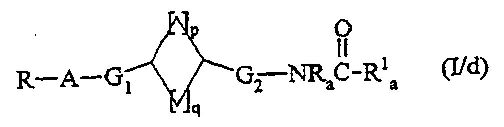 in der R, A, G₁, G₂, Rₐ, R¹ₐ, p und q die oben angegebenen Bedeutungen besitzen,
wobei die Gesamtheit der Verbindungen (I/c) und (I/d) die Verbindung der Formel (I/e) bilden, einem Sonderfall der Verbindungen der Formel (I): 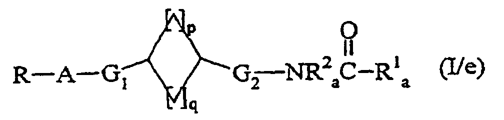 in der R, A, G₁, G₂, R¹ₐ, R²ₐ, p und q die oben angegebenen Bedeutungen besitzen,
welche Verbindung der Formel (I/e) der Einwirkung eines Thionierungsmittels unterworfen werden kann zur Bildung der Verbindung der Formel (I/f), einem Sonderfall der Verbindungen der Formel (I): 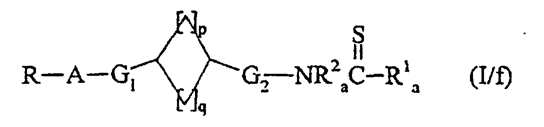 in der R, A, G₁, G₂, R¹ₐ, R²ₐ, p und q die oben angegebenen Bedeutungen besitzen,
- oder der Einwirkung einer Verbindung der Formel (IX) unterworfen werden kann:
Q = C = N - R¹ₐ (IX)
in der Q und R¹ₐ die oben angegebenen Bedeutungen besitzen,
gegebenenfalls gefolgt von der Einwirkung einer Verbindung der Formel (VIII) zur Bildung der Verbindung der Formel (I/g), einem Sonderfall der Verbindungen der Formel (I): 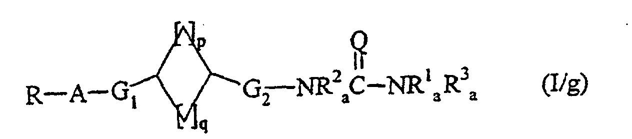 in der R, A, G₁, G₂, R¹ₐ, R²ₐ, R³ₐ, Q, p und q die oben angegebenen Bedeutungen besitzen,
wobei die Verbindungen (I/a) bis (I/g), welche die Gesamtheit der Verbindungen der Formel (I) bilden, mit Hilfe einer klassischen Trennmethode gereinigt werden können, gewünschtenfalls in ihre Additionssalze mit einer pharmazeutisch annehmbaren Säure oder Base überführt werden können und gegebenenfalls mit Hilfe einer klassischen Trennmethode in ihre Isomeren getrennt werden können.

19. Pharmazeutische Zubereitungen enthaltend die Produkte der Formel (I) nach einem der Ansprüche 1 bis 17 oder eines ihrer Additionssalze mit einer pharmazeutisch annehmbaren Säure oder Base in Kombination mit einem oder mehreren pharmazeutisch annehmbaren Trägermaterialien.

20. Pharmazeutische Zubereitungen nach Anspruch 19 geeignet für die Herstellung von Arzneimitteln zur Behandlung von Störungen des melatoninergischen Systems.

## Claims

1. Compounds of formula (I) : 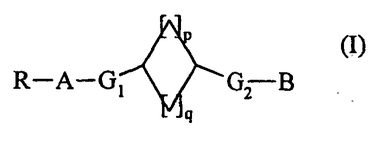 wherein
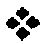 A represents:
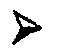 a ring system of formula (II) : 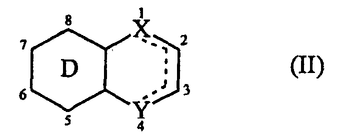 wherein X and Y, which may be identical or different, represent an oxygen atom, a sulphur atom or a CH₂ group,
D represents a benzene ring or a naphthalene,
and the symbol 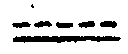 means that the bonds may be single or double, with the proviso that the valency of the atoms is respected,
wherein R substitutes the ring system D or the ring containing X and Y, and G₁ substitutes the ring containing X and Y,
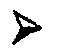 or a ring system of formula (III): 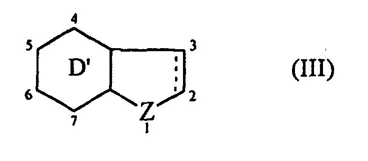 wherein Z represents an oxygen atom, a sulphur atom, a CH₂ group or an NR¹ group (wherein R¹ represents a hydrogen atom, a linear or branched (C₁-C₆)alkyl group, an aryl group or an aryl-(C₁-C₆)alkyl group in which the alkyl moiety is linear or branched),
D' represents a benzene ring or a pyridine ring,
and the symbol 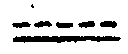 is as defined hereinabove,
wherein R substitutes the D' ring and G₁ substitutes the other ring,
it being understood that the ring systems of formula (II) or (III) may be substituted, as well as by the R and G₁ groups, by from one to three identical or different groups selected from Rₐ, ORₐ, hydroxyl, CORₐ, formyl, COORₐ, carboxyl and OCORₐ,
wherein Rₐ represents a substituted or unsubstituted linear or branched (C₁-C₆)-alkyl group, a substituted or unsubstituted linear or branched (C₂-C₆)alkenyl group, a substituted or unsubstituted linear or branched (C₂-C₆)alkynyl group, a linear or branched polyhalo-(C₁-C₆)alkyl group, a substituted or unsubstituted (C₃-C₈)-cycloalkyl group, a substituted or unsubstituted (C₃-C₈)cycloalkyl-(C₁-C₆)alkyl group in which the alkyl moiety is linear or branched, a substituted or unsubstituted (C₃-C₈)cycloalkenyl group, a substituted or unsubstituted (C₃-C₈)cycloalkenyl-(C₁-C₆)alkyl group in which the alkyl moiety is linear or branched, an aryl group, an aryl-(C₁-C₆)alkyl group in which the alkyl moiety is linear or branched, a heteroaryl group or a heteroaryl-(C₁-C₆)alkyl group in which the alkyl moiety is linear or branched,
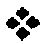 R represents a hydrogen or halogen atom, or a hydroxyl, SH, Rₐ, ORₐ or S(O)ₙRₐ group
wherein n is 0, 1 or 2 and Rₐ is as defined hereinbefore,
or forms, with the carbon atom carrying it and with an adjacent carbon atom, a ring of formula (IV) : 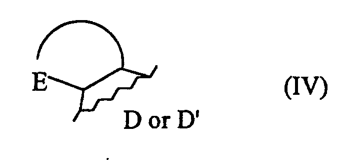 wherein E represents an oxygen atom or an -S(O)ₙ- group wherein n is as defined hereinabove,
wherein the resulting ring contains from 5 to 7 atoms, may contain one or more unsaturated bonds and may be substituted by one or more groups selected from linear or branched (C₁-C₆)alkyl, linear or branched (C₁-C₆)alkoxy, carboxy, linear or branched (C₁-C₆)alkoxy-carbonyl, hydroxy and oxo,
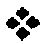 G₁ and G₂, which may be identical or different, represent a single bond or an alkylene chain -(CH₂)ₜ- (wherein t is 1, 2, 3 or 4), unsubstituted or substituted by one or more identical or different radicals selected from hydroxy, carboxy, formyl, Rₐ, ORₐ, COORₐ, CORₐ, (wherein Rₐ is as defined hereinabove) and halogen atoms,
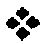 p is 0, 1, 2, 3 or 4,
q is 0, 1, 2, 3 or 4,
with 1 ≤ p+q ≤ 4,
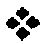 B represents an -NR¹ₐC(Q)R²ₐ, -NR¹ₐC(Q)NR²ₐR³ₐ or -C(Q)NR¹ₐR²ₐ group wherein R¹ₐ, R²ₐ and R³ₐ, which may be identical or different, can have any of the values of Rₐ or may represent a hydrogen atom, and Q represents an oxygen or sulphur atom,
it being understood that:
- the term "substituted" applied to the terms "alkyl", "alkenyl" and "alkynyl" means that those groups are substituted by one or more identical or different radicals selected from hydroxy, linear or branched (C₁-C₆)alkoxy, linear or branched (C₁-C₆)alkyl, linear or branched polyhalo-(C₁-C₆)alkyl and amino groups and halogen atoms,
- the term "substituted" applied to the terms "cycloalkyl", "cycloalkylalkyl", "cycloalkenyl" and "cycloalkenylalkyl" means that the cyclic moiety of those groups is substituted by one or more identical or different radicals selected from hydroxy, linear or branched (C₁-C₆)alkoxy, linear or branched (C₁-C₆)alkyl, linear or branched polyhalo-(C₁-C₆)alkyl and amino groups and halogen atoms,
- "aryl" is understood to mean the groups phenyl, naphthyl or biphenyl, it being possible for those groups to be substituted by one or more identical or different radicals selected from hydroxy, linear or branched (C₁-C₆)alkoxy, linear or branched (C₁-C₆)alkyl, linear or branched polyhalo-(C₁-C₆)alkyl, cyano, carboxy, linear or branched (C₁-C₆)alkoxycarbonyl, nitro and amino groups and halogen atoms,
- "heteroaryl" is understood to mean any mono- or poly-cyclic aromatic group containing from 5 to 10 atoms and containing from 1 to 3 hetero atoms selected from nitrogen, oxygen and sulphur, it being possible for those groups to be substituted by one or more identical or different radicals selected from hydroxy, linear or branched (C₁-C₆)alkoxy, linear or branched (C₁-C₆)alkyl, linear or branched polyhalo-(C₁-C₆)alkyl; cyano, carboxy, linear or branched (C₁-C₆)alkoxy-carbonyl, nitro and amino groups and halogen atoms,
with the proviso that:
- when A represents a naphthalene group which is unsubstituted or substituted by a methoxy group or by a methyl group, G₁ and G₂ simultaneously represent a single bond, and B represents an 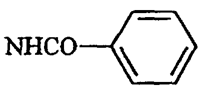 or NHCOMe group, it is not possible to have (q = 0 and p = 4) or (q = 4 and p = 0),
- when G₁ represents a single bond and p+q=1, A cannot represent a naphthalene group substituted by one or more halogen atoms,
- when A represents an indole group substituted in the 2-position by the group 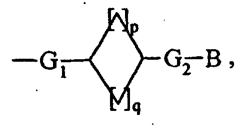 B cannot represent a urea group,
- when A represents an indole group substituted in the 2-position by a carboxyl or alkoxycarbonyl group, p is 1 and q is 0 (or q is 1 and p is 0), and G₁ represents a single bond, B cannot represent a CONHAr group wherein Ar represents an aryl group,
their enantiomers and diastereoisomers, and addition salts thereof with a pharmaceutically acceptable acid or base.

2. Compounds of formula (I) according to claim 1 wherein p is 1, 2, 3 or 4 and q is 0 (or p is 0 and q is 1, 2, 3 or 4), their enantiomers and diastereoisomers, and addition salts thereof with a pharmaceutically acceptable acid or base.

3. Compounds of formula (I) according to claim 1 wherein p is 1 and q is 0 (or p is 0 and q is 1), their enantiomers and diastereoisomers, and addition salts thereof with a pharmaceutically acceptable acid or base.

4. Compounds of formula (I) according to claim 3 **characterised in that** the groups R-A-G₁- and -G₂-B are in a *trans* configuration with respect to one another, their enantiomers and diastereoisomers, and addition salts thereof with a pharmaceutically acceptable acid or base.

5. Compounds of formula (I) according to claim 1 wherein R represents an ORₐ, SRₐ or Rₐ group, their enantiomers and diastereoisomers, and addition salts thereof with a pharmaceutically acceptable acid or base.

6. Compounds of formula (I) according to claim 1 wherein R represents an ORₐ group, their enantiomers and diastereoisomers, and addition salts thereof with a pharmaceutically acceptable acid or base.

7. Compounds of formula (I) according to claim 1 wherein R forms, with the carbon atom carrying it and with an adjacent carbon atom, a ring of formula (IV), their enantiomers and diastereoisomers, and addition salts thereof with a pharmaceutically acceptable acid or base.

8. Compounds of formula (I) according to claim 1 wherein A represents a ring system of formula (II) substituted in the 7-position by R and in the 1- or 2-position by G₁, A being unsubstituted or substituted (as well as by the substituents R and G₁) in the 2- or 3-position, their enantiomers and diastereoisomers, and addition salts thereof with a pharmaceutically acceptable acid or base.

9. Compounds of formula (I) according to claim 1 wherein A represents a ring system of formula (III) substituted in the 5-position by R and in the 3-position by G₁, A being unsubstituted or substituted (as well as by the substituents R and G₁) in the 2-position, their enantiomers and diastereoisomers, and addition salts thereof with a pharmaceutically acceptable acid or base.

10. Compounds of formula (I) according to claim 1 wherein G₁ represents a single bond and G₂ represents a single bond or a CH₂ group, their enantiomers and diastereoisomers, and addition salts thereof with a pharmaceutically acceptable acid or base.

11. Compounds of formula (I) according to claim 1 wherein B represents an -NHCORₐ or -CONHRₐ group, their enantiomers and diastereoisomers, and addition salts thereof with a pharmaceutically acceptable acid or base.

12. Compounds of formula (I) according to claim 1 wherein A is substituted:
- by a group of formula (V) : 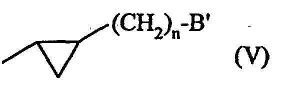
wherein n is 0 or 1 and B' represents an -NHCORₐ or -CONHRₐ group,
- by an ORₐ, SRₐ or Rₐ group, and
- optionally by an alkoxy, aryl or arylalkyl group,
their enantiomers and diastereoisomers, and addition salts thereof with a pharmaceutically acceptable acid or base.

13. Compounds of formula (I) according to claim 1 wherein A represents a naphthalene, a chroman or a benzochroman,
- substituted in the 1- or 2-position (formula II) by a group of formula (V) : 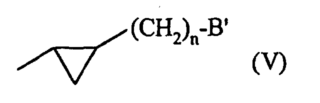
wherein n is 0 or 1 and B' represents an -NHCORₐ or -CONHRₐ group,
- substituted in the 7-position (formula II) by an ORₐ, SRₐ or Rₐ group, and
- optionally substituted in the 3-position (formula II) by an aryl or arylalkyl group, or in the 2-position (formula II) by an alkoxy group,
their enantiomers and diastereoisomers, and addition salts thereof with a pharmaceutically acceptable acid or base.

14. Compounds of formula (I) according to claim 1 wherein A represents a benzothiophene, a benzofuran, an indole or an azaindole,
- substituted in the 3-position (formula III) by a group of formula (V) : 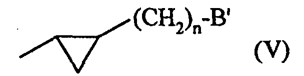
wherein n is 0 or 1 and B' represents an -NHCORₐ or -CONHRₐ group,
- substituted in the 5-position (formula III) by an ORₐ, SRₐ or Rₐ group, and
- optionally substituted in the 2-position (formula III) by an aryl or arylalkyl group, their enantiomers and diastereoisomers, and addition salts thereof with a pharmaceutically acceptable acid or base.

15. Compounds of formula (I) according to claim 1 wherein A represents a naphthalene,
- substituted in the 1-position by a group of formula (V) : 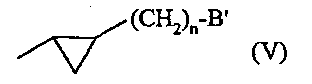
wherein n is 0 or 1 and B' represents an -NHCORₐ or -CONHRₐ group,
- substituted in the 7-position by an ORₐ group, and
- optionally substituted in the 3-position by an aryl or arylalkyl group, or in the 2-position by an ORₐ group,
their enantiomers and diastereoisomers, and addition salts thereof with a pharmaceutically acceptable acid or base.

16. Compounds of formula (I) according to claim I which are :
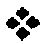 N-[2-(7-methoxy-1-naphthyl)-1-cyclopropyl]acetamide
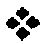 N-[2-(7-methoxy-1-naphthyl)-1-cyclopropyl]propanamide
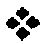 N-[2-(7-methoxy-1-naphthyl)-1-cyclopropyl]butanamide
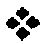 N-[2-(7-methoxy-1-naphthyl)-1-cyclopropyl]cyclopropanecarboxamide
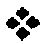 N-[2-(2,7-dimethoxy-1-naphthyl)-1-cyclopropyl]acetamide
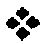 N-[2-(2,7-dimethoxy-1-naphthyl)-1-cyclopropyl]propanamide
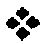 N-[2-(2,7-dimethoxy-1-naphthyl)-1-cyclopropyl]butanamide
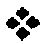 N-[2-(2-methoxy-1-naphthyl)-1-cyclopropyl]acetamide
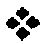 N-[2-(2-methoxy-1-naphthyl)-1-cyclopropyl]propanamide
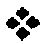 N-[2-(2-methoxy-1-naphthyl)-1-cyclopropyl]butanamide
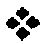 N-[2-(2-methoxy-1-naphthyl)-1-cyclopropylmethyl]acetamide
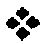 N-[2-(2-methoxy-1-naphthyl)-1-cyclopropylmethyl]propanamide
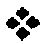 N-[2-(2-methoxy-1-naphthyl)-1-cyclopropylmethyl]butanamide
their enantiomers and diastereoisomers, and addition salts thereof with a pharmaceutically acceptable acid or base.

17. Compounds of formula (I) according to claim 15 **characterised in that** the groups -(CH₂)ₙ-B' and R-A-G₁- are in a *trans* configuration with respect to one another, their enantiomers and diastereoisomers, and addition salts thereof with a pharmaceutically acceptable acid or base.

18. Process for the preparation of compounds of formula (I) according to claim 1 **characterised in that** there is used as starting material a compound of formula (VI) : 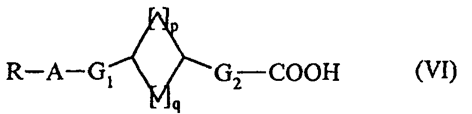 wherein R, A, G₁, G₂, p and q are as defined hereinabove,
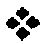 which is subjected, after activation to the acid chloride or in the presence of a coupling agent, to the action of an amine HNR¹ₐR²ₐ, wherein R¹ₐ and R²ₐ are as defined hereinabove to yield a compound of formula (I/a), a particular case of the compounds of formula (I) : 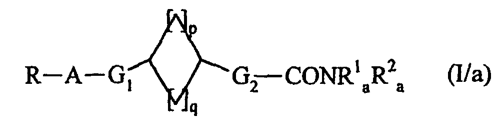 wherein R, A, G₁, G₂, R¹ₐ, R²ₐ, p and q are as defined hereinabove,
which may be subjected to the action of a thionisation agent to obtain a compound of formula (I/b), a particular case of the compounds of formula (I), 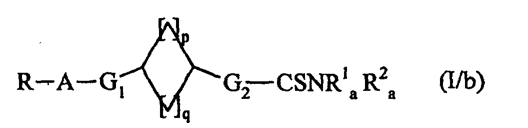 wherein R, A, G₁, G₂, R¹ₐ, R²ₐ, p and q are as defined hereinabove,
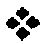 or subjected, after conversion to the corresponding azide, to a Curtius rearrangement to yield, after hydrolysis, an amine of formula (VII) : 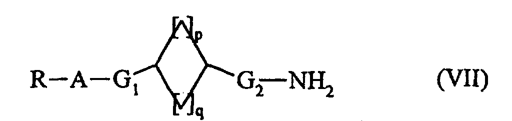 wherein R, A, G₁, G₂, p and q are as defined hereinabove,
which is either:
- reacted with an acyl chloride ClCOR¹ₐ or the corresponding mixed or symmetric anhydride wherein R¹ₐ is as defined hereinabove to yield a compound of formula (I/c), a particular case of the compounds of formula (I): 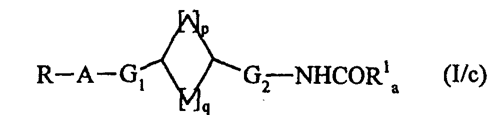 wherein R, A, G₁, G₂, R¹ₐ, p and q are as defined hereinabove,
followed optionally by the action of a compound of formula (VIII) :
Rₐ―J (VIII)
wherein Rₐ is as defined hereinabove and J represents a leaving group, such as a halogen atom or a tosyl group,
to yield a compound of formula (I/d), a particular case of the compounds of formula (I): 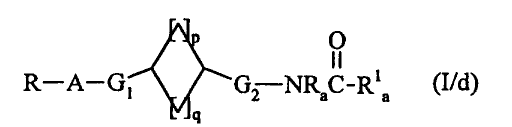 wherein R, A, G₁, G₂, Rₐ, R¹ₐ, p and q are as defined hereinabove,
the totality of the compounds (I/c) and (I/d) constituting the compounds of formula (I/e), a particular case of the compounds of formula (I) : 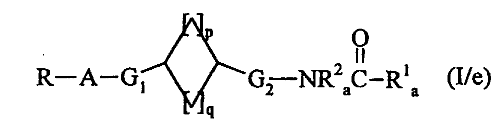 wherein R, A, G₁, G₂, R¹ₐ, R²ₐ, p and q are as defined hereinabove,
which compounds of formula (I/e) may be subjected to the action of a thionisation agent to yield a compound of formula (I/f), a particular case of the compounds of formula (I) : 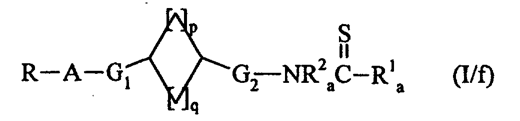 wherein R, A, G₁, G₂, R¹ₐ, R²ₐ, p and q are as defined hereinabove,
or
- subjected to the action of a compound of formula (IX) :
Q=C=N-R¹ₐ (IX)
wherein Q and R¹ₐ are as defined hereinabove, followed optionally by the action of a compound of formula (VIII) to yield a compound of formula (I/g), a particular case of the compounds of formula (I) : 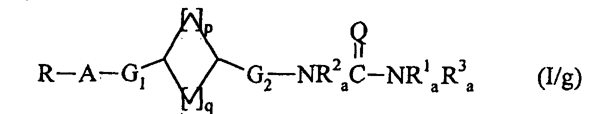 wherein R, A, G₁, G₂, R¹ₐ, R²ₐ, R³ₐ, Q, p and q are as defined hereinabove,
which compounds (I/a) to (I/g) constitute the totality of the compounds of formula (I) and may be purified according to a conventional separation technique, are converted, if desired, into their addition salts with a pharmaceutically acceptable acid or base, and optionally separated into their isomers according to a conventional separation technique.

19. Pharmaceutical compositions comprising the products of formula (I) according to any one of claims 1 to 17 or an addition salt thereof with a pharmaceutically acceptable acid or base in combination with one or more pharmaceutically acceptable excipients.

20. Pharmaceutical compositions according to claim 19 for use in the manufacture of medicaments for treating disorders of the melatoninergic system.
